# EUROPEAN PATENT SPECIFICATION

(11) **EP 0 441 900 B1**
(45) Date of publication and mention of the grant of the patent: **17.07.1996**
(21) Application number: 90901161.1
(22) Date of filing: 09.11.1989
(51) Int. Cl.: C12N 15/26, C07K 14/54, A61K 38/20

(54) **NATURAL KILLER STIMULATORY FACTOR**
NATÜRLICHER KILLERZELLEN-STIMULATIONSFAKTOR
FACTEUR STIMULATEUR DE CELLULES CYTOTOXIQUES NATURELLES

(30) Priority: 10.11.1988 US 269945; 07.02.1989 US 307817
(43) Date of publication of application: 21.08.1991
(73) Proprietor: GENETICS INSTITUTE, INC., Cambridge, Massachusetts 02140 (US); THE WISTAR INSTITUTE, Philadelphia, PA 19104 (US)
(72) Inventor: TRINCHIERI, Giorgio, Wynnewood, PA 19104 (US); PERUSSIA, Bice, Philadelphia, PA 19146 (US); KOBAYASHI, Michiko, Kanagawa Yokohama (JP); CLARK, Steven, C., Winchester, MA 01890 (US); WONG, Gordon, G., Jamaica Plain, MA 02130 (US); HEWICK, Rodney, M., Lexington, MA 02173 (US)
(74) Representative: VOSSIUS & PARTNER
(86) International application number: US8905027
(87) International publication number: WO9005147

(56) References cited:
- EP-A- 0 357 067
- US-A- 4 473 555
- Journal of Biological Response Modifiers, Vol. 4, 1985 James E. Talmadge: "Immunoregulation and Immunostimulation of Murine Lymphocytes by Recombinant Human Interleukin-2 ",
- PROGRESS IN IMMUNOLOGY V, Vol. 5, 1983 Gy.G. Petranyi et al: "Natural Killer Cells in Man:Genetic and Other Factors Regulating Their Activiy ", see page 1169, pages 1174-1175, pages 1178-1181.
- SCIENCE, Vol. 214, 1981 Ronald B. Herberman et al. Pages 24-30
- Dialog Information Services, File 154, Medline 89-20. Dialog accession no. 89361252 M Kobayashi et al. J Exp Med, Sep 1 1989, 170(3) p 827-45

## Description

The present invention relates to a novel cytokine that stimulates the function of natural killer cells and other cells of the immune system, and to processes for obtaining the natural killer stimulatory factor in homogeneous form and producing it by recombinant genetic engineering techniques.

### Background of the Invention

Natural killer (NK) cells are a subset of lymphocytes active in the immune system and representing an average 15% of mononuclear cells in human peripheral blood [G. Trinchieri and B. Perussia, Lab. Invest., 50:489 (1984)]. Among the surface markers used to identify human NK cells is a receptor binding with low affinity to the Fc fragment of IgG antibodies, such as Fc-gamma receptor III or CD16 antigen [B. Perussia et al, J. Immunol., 133:180 (1984)]. NK cells have been demonstrated to play an important role in vivo in the defense against tumors, tumor metastases, virus infection, and to regulate normal and malignant hematopoiesis.

A growing family of regulatory proteins that deliver signals between cells of the immune system has been identified. These regulatory molecules are known as cytokines. Many of the cytokines have been found to control the growth, development and biological activities of cells of the hematopoietic and immune systems. These regulatory molecules include all of the colony-stimulating factors (GM-CSF, G-CSF, M-CSF, and multi CSF or interleukin-3), the interleukins (IL-1 through IL-7), the interferons (alpha, beta and gamma), the tumor necrosis factors (alpha and beta) and leukemia inhibitory factor (LIF). These cytokines exhibit a wide range of biological activities with target cells from bone marrow, peripheral blood, fetal liver, and other lymphoid or hematopoietic organs. See, e.g., G. Wong and S. Clark, Immunology Today, 9(5):137 (1988).

The biochemical and biological identification and characterization of certain cytokines was hampered by the small quantities of the naturally occurring factors available from natural sources, e.g., blood and urine. Many of the cytokines have recently been molecularly cloned, heterologously expressed and purified to homogeneity. [D. Metcalf, "The Molecular Biology and Functions of the Granulocyte-Macrophage Colony Stimulating Factors," Blood, 67(2):257-267 (1986).] Among these cytokines are gamma interferon, human and murine GM-CSF, human G-CSF, human CSF-1 and human and murine IL-3. Several of these purified factors have been found to demonstrate regulatory effects on the hematopoietic and immune systems in vivo, including GM-CSF, G-CSF, IL-3 and IL-2.

There remains a need in the art for additional proteins purified from their natural sources or otherwise produced in homogeneous form, which are capable of stimulating or enhancing immune responsiveness and are suitable for pharmaceutical use.

### Summary of the Invention

In one aspect the present invention provides a novel human natural killer stimulatory factor, called NKSF, comprising a subunit encoded by a DNA sequence comprising a DNA sequence selected from
(a) the DNA sequence shown in Table I;
(b) DNA sequences which hybridise to the DNA sequence of (a);
(c) allelic variations, analogues, derivatives or fragments of the DNA sequence of (a) or (b); or
(d) DNA sequences which differ in codon sequence due to the degeneracy of the genetic code;
wherein the NKSF protein containing said subunit retains NKSF activity and is active in a gamma interferon induction assay. Active NKSF has an apparent molecular weight of approximately 70 kd. Pure preparations of NKSF reveal the presence of two polypeptides, which are contemplated as subunits which, when associated, yield active NKSF. It is presently speculated that NKSF is a heterodimer formed by association of both the larger and smaller subunits through one or more disulfide bonds. This apparent heterodimeric structure could be generated by association of the two individual subunits or by proteolytic cleavage of a single precursor polypeptide, e.g. as with insulin. Alternatively, it is possible that the active form of NKSF is a homodimer of the larger subunit or a homodimer of the smaller subunit.

The active approximately 70-80 kd NKSF is further characterized by containing all or a portion of the amino acid sequence of Table I below. Additionally, one or more of the following sequences of amino acids (in single letter code) is present in the primary sequence of either the larger or smaller of the NKSF subunits. ( ) indicate an amino acid not positively identified.

L-T-I-Q-V

K-Y-E-N-Y-T

I-W-E-L-K

L-M-D-P-K

V-M-S-Y-L-N-A

(A)-V-S-N-M-L-Q-K

N-A-S-I-S-V

T-F-L-R

D-I-I-K-P-D-P-P-K.

The larger subunit polypeptide of NKSF is characterized by having an apparent molecular weight of 40kd. This subunit is further characterized by having the following amino terminal sequence: I-W-E-L-K-K-D-V-Y-V-V-E-L-D-W-Y-P-D-A-P-G-E-M. This larger polypeptide is further characterized by containing all or a portion of the longer cloned sequence as disclosed in Table I below.

The smaller polypeptide subunit of NKSF is characterized by an apparent molecular weight of approximately 30-35 kd and is further characterized by having the following amino terminal sequence: (X)-N-L-P-V-A-(P)-P-D-P-(S or T)-M-F-P. The (X) indicates that the first residue of the sequence of the smaller subunit could not be determined.

NKSF displays biological activity in inducing the production of gamma interferon in vitro by human peripheral blood lymphocytes (PBLs). In homogeneous form, NKSF is characterized by a specific activity of greater than 1X10⁷ dilution units per milligram in the gamma interferon induction assay, described in detail below.

In addition to the induction of gamma interferon in PBLs, NKSF demonstrates the following biological activities:
(1) biological activity in a granulocyte-macrophage colony stimulating factor (GM-CSF) inducing assay with PBLs;
(2) biological activity in activating Natural Killer (NK) cells to kill leukemia and tumor-derived cells;
(3) biological activity in a tumor necrosis factor (TNF) inducing assay with phytohemagglutinin (PHA)-activated T lymphocytes; and
(4) co-mitogenic activity with peripheral blood T lymphocytes.

Another aspect of the invention includes DNA sequences comprising cDNA sequences coding on expression for a human NKSF polypeptide, a human NKSF larger subunit polypeptide, and a human NKSF smaller subunit polypeptide. Such sequences include a sequence of nucleotides encoding one or more of the subunits and peptide sequences described above.

Also provided by the present invention is a vector containing a DNA sequence encoding NKSF or a subunit of NKSF in operative association with an expression control sequence. Host cells transformed with such vectors for use in producing recombinant NKSF or its recombinant subunits are also provided by the present invention.

As still a further aspect of the present invention, there is provided recombinant NKSF protein. This protein is free from other mammalian proteinaceous materials and is characterized by being encoded by a DNA sequence encoding one or more of the above-described subunits or peptide fragments containing one or more of the above-described physical, biochemical or biological activities or characteristics.

Another aspect of this invention provides pharmaceutical compositions containing a therapeutically effective amount of homogeneous or recombinant NKSF, or an effective amount of one or both of the subunits of NKSF, or of one or more of the peptide fragments thereof.

In a preferred embodiment said pharmaceutical compositions further comprise at least one other cytokine, hematopoietin, growth factor, or antibody capable of binding to the FC portion of NK cells. More preferably, said cytokine is IL-1, IL-2 or IL-6.

These pharmaceutical compositions may be employed in methods for treating cancer, and other disease states responsive to the enhanced presence of gamma interferon and GM-CSF production. Thus, generally this factor may be employed in the treatment of diseases characterized by a deficiency in the number or level of activity of hematopoietic cells.

Still a further aspect of the present invention is a process for producing homogeneous NKSF, or a subunit thereof from a human cell line producing NKSF or a subunit thereof in admixture with other proteins and polypeptides. This process of production provided by the present invention includes culturing selected cells capable of producing NKSF, its subunits, or peptide fragments thereof to obtain conditioned medium and purifying the conditioned medium through five primary purification steps.

The vectors and transformed cells of the invention are employed in another aspect, a novel process for producing recombinant human NKSF protein, a subunit thereof or peptide fragments thereof. In this process a cell line transformed with a DNA sequence encoding on expression NKSF protein, a subunit thereof or a peptide fragment thereof in operative association with an expression control sequence therefor is cultured. This claimed process may employ a number of known cells as host cells for expression of the polypeptide. Presently preferred cell lines are mammalian cell lines and bacterial cells.

Other aspects and advantages of the present invention will be apparent upon consideration of the following detailed description of preferred embodiments thereof.

### Detailed Description of the Invention

The novel human natural killer cell stimulatory factor, NKSF, provided by the present invention is a protein comprising a subunit encoded by a DNA sequence comprising a DNA sequence selected from
(a) the DNA sequence shown in Table I;
(b) DNA sequences which hybridise to the DNA sequence of (a);
(c) allelic variations, analogues, derivatives or fragments of the DNA sequence of (a) or (b); or
(d) DNA sequences which differ in codon sequence due to the degeneracy of the genetic code;
wherein the NKSF protein containing said subunit retains NKSF activity and is active in a gamma interferon induction assay.

Natural killer stimulatory factor has an apparent molecular weight of approximately 70-80 kd as determined by sodium dodecylsulfate polyacrylamide gel electrophoresis (SDS-PAGE) under non-reducing conditions. This 70-80 kd peptide is active in a gamma interferon induction assay.

Under reducing conditions in SDS-PAGE, the 70-80 kd band yields two smaller subunits with apparent molecular weights of 40 kd (larger subunit) and approximately 30-35 kd (smaller subunit). For both subunits, the biological activity in the same gamma interferon induction assay is substantially lost compared to that of the native 70-80 kd species. The amino terminal sequences identified above were determined from the 40 kd reduced species and the 30-35 kd reduced species believed to be the subunits of the NKSF heterodimer. It is presently believed that NKSF is a disulfide-bonded heterodimer of the larger and smaller subunits.

NKSF is, at least in part, an anionic glycoprotein. Under isoelectric focusing, two species of the NKSF are observed having isoelectric points of 4.3 and 4.8. It is presently speculated that the two species differ in glycosylation patterns.

NKSF is primarily characterized by biological activity in the gamma interferon induction assay described in detail in Example 8 below. Among its other biological activities is included the ability to induce GM-CSF production by human peripheral blood lymphocytes. [See, e.g., published PCT application WO86/00639 for additional information on GM-CSF]. NKSF also has an enhancing effect on the mitogenic activity of various mitogens, such as lectins and phorbol diesters, on peripheral blood T lymphocytes and has a growth promoting effect on activated human tonsillar B cells.

NKSF has also been observed to enhance NK cell functions to kill leukemia and tumor-derived cells in vitro using a spontaneous cell cytotoxicity assay and an antibody dependent cell cytotoxicity (ADCC) assay.

Briefly, in a spontaneous cell cytotoxicity assay, human peripheral blood lymphocytes or purified NK cells are incubated in the presence of NKSF for a period of 8 to 18 hours. Lymphocytes and NK cells are then assayed in a standard ⁵¹Cr-release assay for their ability to lyse target cells such as leukemia cell lines, tumor-derived cell lines, or virus-infected fibroblasts. NKSF dramatically increases the ability of NK cells to lyse such target cells at a level comparable to that obtained with interferon alpha and IL-2, well known activators of NK cell cytotoxic activity [See, e.g., G. Trinchieri et al, J. Exp. Med., 147:1314 (1978) and G. Trinchieri et al, J. Exp. Med., 160:1146 (1984)].

In an ADCC assay target cancer cells are coated with antibodies capable of binding to the Fc receptor on NK cells, e.g., IgG₂ₐ, IgG₃ and the like. In preliminary assays, the presence of NKSF appears to enhance the killing activity of the NK cells for the coated tumor cells in ADCC. [See, e.g., L. M. Weiner et al, Cancer Res., 48:2568-2573 (1988); P. Hersey et al, Cancer Res., 46:6083-6090 (1988); and C. J. Hansik et al, Proc. Natl. Acad. Sci., 83:7893-97 (1986) for additional information on ADCC.]

Preliminary analysis of NKSF in a B-cell growth factor assay using normal human B cells stimulated with goat anti-human IgM antibody (anti-µ) coupled to beads indicates that NKSF may also be characterized by B cell growth factor activity. In this assay the antibody directed against the IgM immunoglobulin on the surface of the B cell activates the B cell and causes it to become responsive to B cell growth factors. [See, C-T K. Tseng et al, J. Immunol., 140:2305-2311 (1988)]. Such antibodies are commercially available.

NKSF was originally detected in the conditioned medium of the human cell line, RPMI 8866, a commercially available cell line [University of Pennsylvania Cell Center] which produces a mixture of lymphokines. This factor may also be produced by other Epstein Barr virus-transformed lymphoblastoid cell lines or from other human cell lines. The purification technique employed in obtaining NKSF from cells which naturally produce it, uses the following steps. These steps include purification through an ion exchange column, e.g., QAE Zeta preparative cartridge [LKB Pharmacia], which indicates that the NKSF protein is anionic. The second purification step is a lentil lectin column which demonstrates that NKSF is, at least in part, a glycoprotein. The eluate from the lentil lectin column is further purified through a hydroxylapatite column, followed by a heparin sepharose column and a fast protein liquid chromatography (FPLC) Mono-Q column. The NKSF from RPMI 8866 eluted as a single peak in each of the three latter columns. A remaining protein contaminant of about 37 kd is removed by gel filtration chromatography alone or reverse phase HPLC and gel filtration chromatography. The resulting purified homogeneous NKSF was assayed for biological activity in the gamma interferon induction assay of Example 8 and demonstrated a specific activity of greater than 1X10⁷ dilution units per milligram.

Thus, the homogeneous NKSF may be obtained by applying the above purification procedures, which are described in detail in Example 2, to the conditioned medium of RPMI 8866 or other sources of human NKSF. The RPMI 8866 cell line produces the factor spontaneously, but the level of production can be enhanced by treating the cell line with phorbol esters, such as phorbol dibutyrate. The cells deprived of serum for 48 hours still produce NKSF along with other lymphokines. Procedures for culturing RPMI 8866 (see Example 1) or another cell source of NKSF are known to those of skill in the art.

NKSF or one or both of its subunits or peptide fragments thereof may also be produced via recombinant techniques. To obtain the DNA sequence for cloned NKSF or one or both of its subunits, tryptic digests of the homogeneous polypeptide are prepared. For example, nine tryptic digests found in a subunit of NKSF are identified below: 
L-T-I-Q-V
K-Y-E-N-Y-T
L-M-D-P-K
I-W-E-L-K
V-M-S-Y-L-N-A
(A)-V S-N-M-L-Q-K
N-A-S-I-S-V
T-F-L-R
D-I-I-K-P-D-P-P-K.
 Additionally, the amino terminal sequences of the larger and smaller subunits of NKSF are identified above.

Oligonucleotide probes are synthesized using the genetic code to predict all possible sequences that encode the amino acid sequences of these tryptic digestion products of NKSF. The same procedure may be followed by constructing probes from the above-identified amino terminal sequences of the two subunits of NKSF. The NKSF gene or the subunit genes can be identified by using these probes to screen a human genomic library. Alternatively, the mRNA from RPMI 8866 or another cell source of NKSF can be used to make a cDNA library which can be screened with the probes to identify the cDNAs encoding the NKSF polypeptide or the polypeptides of its large and small subunits. Once the cDNAs are identified, they can be co-introduced into any one of a variety of expression vectors to make an expression system for NKSF, or one or both of its subunits.

By such use of recombinant techniques, DNA sequences encoding the NKSF polypeptide or the polypeptides of its large and/or small subunit are obtained which contain DNA sequences encoding one or more of the tryptic fragments or the amino terminal sequences identified above.

One such NKSF clone, named pNK-6, has at least the following DNA and amino acid sequences and codes for all or a portion of the larger NKSF subunit:

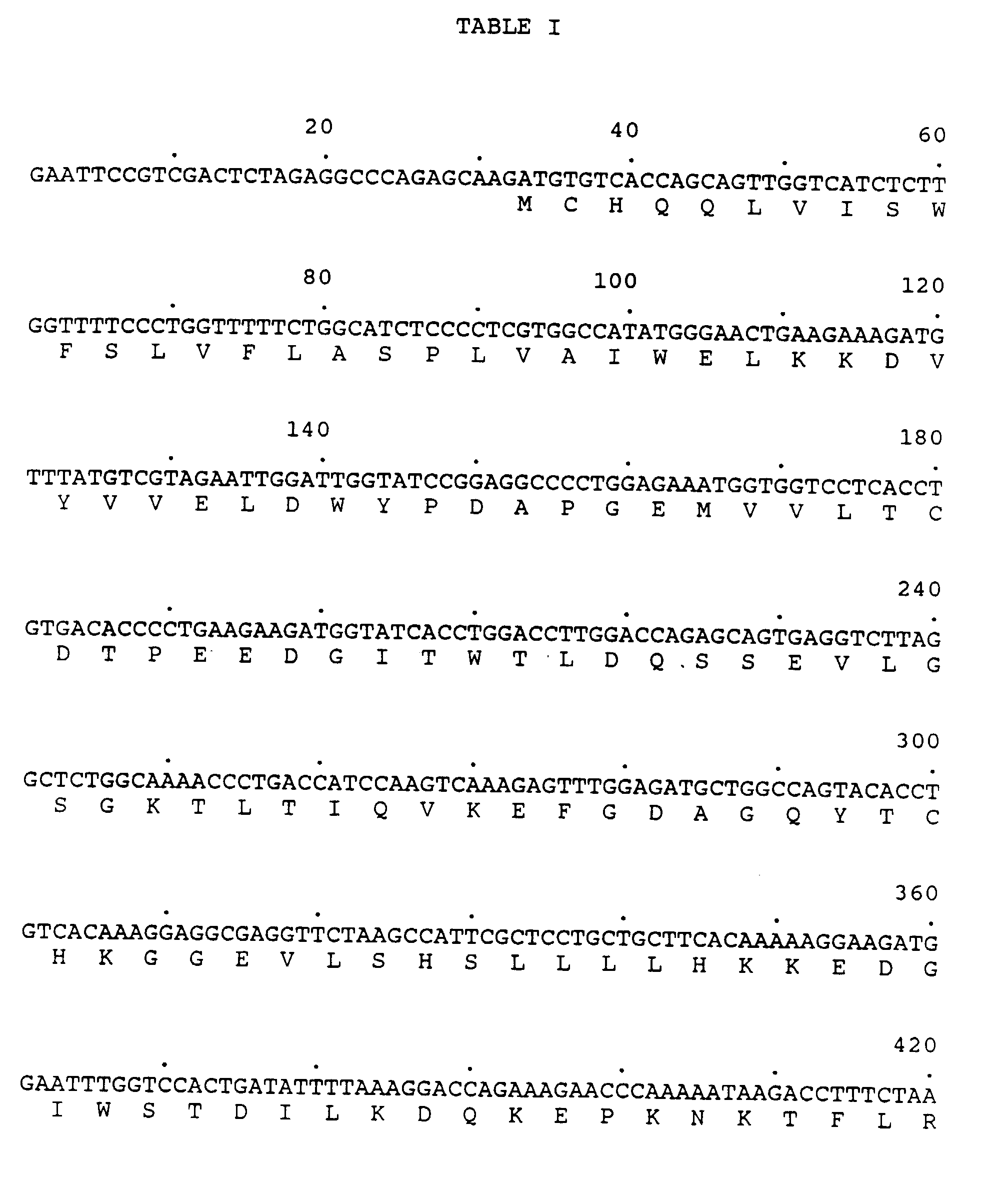

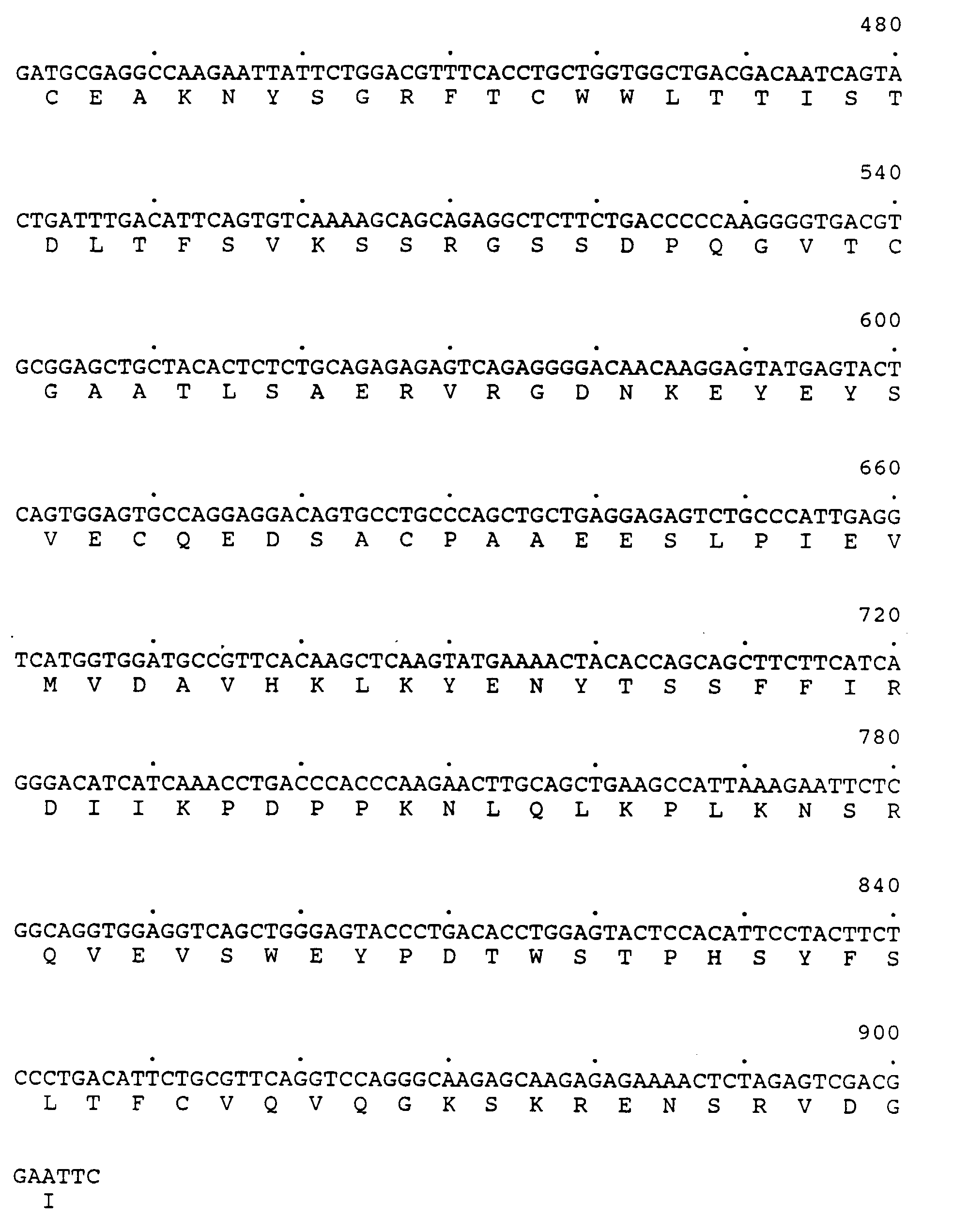

This sequence cloned in plasmid pNK-6 was deposited with the American Type Culture Collection, 12301 Parklawn Drive, Rockville, Maryland on February 3, 1989 under accession number 40545. Additional clones are obtained, and when sequenced, are expected to provide the carboxy-terminal sequence of the larger subunit, and/or the sequence of the smaller subunit of NKSF.

Allelic variations of DNA sequences encoding the peptide sequences and the larger subunit described above are also included in the present invention as well as analogs or derivatives thereof.

Thus the present invention also encompasses these novel DNA sequences, free of association with DNA sequences encoding other primate proteins, and coding on expression for NKSF polypeptides, including those of its large and small subunits. These DNA sequences include those containing one or more of the above DNA sequences and sequences encoding one or more of the above-identified peptide sequences and those sequences which hybridize under stringent hybridization conditions [see, T. Maniatis et al, Molecular Cloning (A Laboratory Manual), Cold Spring Harbor Laboratory (1982), pages 387 to 389] to the DNA sequences. An example of one such stringent hybridization condition is hybridization at 4XSSC at 65°C, followed by a washing in 0.1XSSC at 65°C for an hour. Alternatively an exemplary stringent hybridization condition is in 50% formamide, 4XSSC at 42°C.

DNA sequences which hybridize to the sequences for NKSF or its subunits under relaxed hybridization conditions and which code on expression for NKSF peptides having NKSF biological properties also encode novel NKSF polypeptides. Examples of such non-stringent hybridization conditions are 4XSSC at 50°C or hybridization with 30-40% formamide at 42°C. For example, a DNA sequence which shares regions of significant homology, e.g., sites of glycosylation or disulfide linkages, with the sequences of NKSF and encodes a protein having one or more NKSF biological properties clearly encodes a NKSF polypeptide even if such a DNA sequence would not stringently hybridize to the NKSF sequences.

Similarly, DNA sequences which code for NKSF polypeptides having the sequence of NKSF, but which differ in codon sequence due to the degeneracies of the genetic code or allelic variations (naturally-occurring base changes in the species population which may or may not result in an amino acid change) are also encompassed by this invention. Variations in the DNA sequence of NKSF which are caused by point mutations or by induced modifications to enhance the activity, half-life or production of the polypeptides encoded thereby are also encompassed in the invention.

NKSF polypeptides may also be produced by known conventional chemical synthesis. Methods for constructing the polypeptides of the present invention by synthetic means are known to those of skill in the art. The-synthetically-constructed NKSF polypeptide sequences, by virtue of sharing primary, secondary, or tertiary structural and conformational characteristics with NKSF polypeptides may possess NKSF biological properties in common therewith. Thus, they may be employed as biologically active or immunological substitutes for natural, purified NKSF polypeptides in therapeutic and immunological processes.

The NKSF polypeptides provided herein also include factors having sequences similar to those of purified homogeneous and recombinant NKSF protein, or the subunit polypeptides, but into which modifications are naturally provided or deliberately engineered.

Modifications in the peptides or DNA sequences can be made by one skilled in the art using known techniques. Modifications of interest in the NKSF sequences may include the replacement, insertion or deletion of a selected amino acid residue in the coding sequences. Mutagenic techniques for such replacement, insertion or deletion are well known to one skilled in the art. [See, e.g., United States patent 4,518,584.]

Other specific mutations of the sequences of the NKSF polypeptide or the subunit polypeptides described herein may involve modifications of a glycosylation site. The absence of glycosylation or only partial glycosylation results from amino acid substitution or deletion at any asparagine-linked glycosylation recognition site or at any site of the molecule that is modified by addition of O-linked carbohydrate. An asparagine-linked glycosylation recognition site comprises a tripeptide sequence which is specifically recognized by appropriate cellular glycosylation enzymes. These tripeptide sequences are either asparagine-X-threonine or asparagine-X-serine, where X is usually any amino acid. A variety of amino acid substitutions or deletions at one or both of the first or third amino acid positions of a glycosylation recognition site (and/or amino acid deletion at the second position) results in non-glycosylation at the modified tripeptide sequence.

Expression of such altered nucleotide sequences produces variants which are not glycosylated at that site.

Other analogs and derivatives of the sequence of NKSF or of its subunits which would be expected to retain NKSF activity in whole or in part may also be easily made by one of skill in the art given the disclosures herein. One such modification may be the attachment of polyethylene glycol onto existing lysine residues or the insertion of a lysine residue into the sequence by conventional techniques to enable the attachment. Such modifications are believed to be encompassed by this invention.

The present invention also provides a method for producing NKSF polypeptides. The method of the present invention involves culturing a suitable cell or cell line, which has been transformed with a DNA sequence coding on expression for an NKSF polypeptide, including a subunit polypeptide, under the control of known regulatory sequences. Suitable cells or cell lines may be mammalian cells, such as Chinese hamster ovary cells (CHO) or 3T3 cells. The selection of suitable mammalian host cells and methods for transformation, culture, amplification, screening and product production and purification are known in the art. See, e.g., Gething and Sambrook, Nature, 293:620-625 (1981), or alternatively, Kaufman et al, Mol. Cell. Biol., 5(7):1750-1759 (1985) or Howley et al, U. S. Patent 4,419,446. Coexpression of two different cDNAs simultaneously in CHO cells has been described, for example, in published PCT International Application WO88/08035. Other suitable mammalian cell lines, are the monkey COS-1 cell line, and the CV-1 cell line, originally developed at the Wistar Institute, Philadelphia, Pennsylvania.

Similarly useful as host cells suitable for the present invention are bacterial cells. For example, various strains of E. coli (e.g., HB101, MC1061 and strains used in the following examples) are well-known as host cells in the field of biotechnology. Various strains of B. subtilis, Pseudomonas, other bacilli and the like may also be employed in this method.

Many strains of yeast cells known to those skilled in the art are also available as host cells for expression of the polypeptides of the present invention. Additionally, where desired, insect cells may be utilized as host cells in the method of the present invention. See, e.g. Miller et al, Genetic Engineering, 8:277-298 (Plenum Press 1986) and references cited therein.

The present invention also provides vectors for use in the method of expression of novel NKSF polypeptides. These vectors contain che novel NKSF DNA sequences which code for NKSF polypeptides of the invention, including the subunit polypeptides. Alternatively, vectors incorporating modified sequences as described above are also embodiments of the present invention and useful in the production of NKSF polypeptides. The vector employed in the method also contains selected regulatory sequences in operative association with the DNA coding sequences of the invention and capable of directing the replication and expression thereof in selected host cells.

Thus NKSF, purified to homogeneity from cell sources or produced recombinantly or synthetically, may be used in a pharmaceutical preparation or formulation to treat cancer or other disease states which respond to enhanced NK cell activity or increased in vivo production of gamma interferon or GM-CSF. Such pathological states may result from disease, exposure to radiation or drugs, and include for example, leukopenia, bacterial and viral infections, anemia, B cell or T cell deficiencies including immune cell or hematopoietic cell deficiency following a bone marrow transplantation. Therapeutic treatment of cancer and other diseases with these NKSF polypeptide compositions may avoid undesirable side effects caused by treatment with presently available drugs.

It may also be possible to employ one or both of the subunit polypeptides of NKSF or peptide fragments thereof in such pharmaceutical formulations.

The polypeptides of the present invention may also be employed, alone or in combination with other cytokines, hematopoietins, interleukins, growth factors or antibodies in the treatment of cancer or other diseases states. Other uses for these novel polypeptides are in the development of monoclonal and polyclonal antibodies generated by standard methods for diagnostic or therapeutic use.

Therefore, another aspect of the invention are therapeutic compositions for treating the conditions referred to above. Such compositions comprise a therapeutically effective amount of the NKSF protein or a subunit polypeptide or therapeutically effective fragment thereof of the present invention in admixture with a pharmaceutically acceptable carrier. This composition can be systemically administered parenterally. Alternatively, the composition may be administered intravenously. If desirable, the composition may be administered subcutaneously. When systematically administered, the therapeutic composition for use in this invention is in the form of a pyrogen-free, parenterally acceptable aqueous solution. The preparation of such a pharmaceutically acceptable protein solution, having due regard to pH, isotonicity, stability and the like, is within the skill of the art.

The dosage regimen involved in a method for treating the above-described conditions will be determined by the attending physician considering various factors which modify the action of drugs, e.g. the condition, body weight, sex and diet of the patient, the severity of any infection, time of administration and other clinical factors. Generally, the daily regimen should be in the range of 1-1000 micrograms of NKSF protein or subunit thereof or 50 to 5000 units (i.e., one unit per ml being the concentration of protein which leads to half maximal stimulation in the gamma interferon induction assay) of protein per kilogram of body weight.

The therapeutic compositions of the present invention may also include co-administration with other human factors. Exemplary cytokines or hematopoietins for such use include the known factors IL-1, IL-2 and IL-6 particularly. [See, e.g., PCT publications WO85/05124, and WO88/00206; and European patent application 0,188,864.] Other potential candidates for participation in NKSF therapy may also include IL-4, G-CSF, CSF-1, GM-CSF, IL-3 or erythropoietin. Growth factors like B cell growth factor, B cell differentiation factor, or eosinophil differentiation factors may also prove useful in co-administration with NKSF.

Similarly, administration of NKSF or a subunit or fragment thereof with or prior to administration of an antibody capable of binding to the Fc receptor on NK cells may enhance ADCC therapy directed against tumors. The dosage recited above would be adjusted to compensate for such additional components in the therapeutic composition. Progress of the treated patient can be monitored by conventional methods.

The following examples illustratively describe the purification and characteristics of homogeneous human NKSF and other methods and products of the present invention. These examples are for illustration and do not limit the scope of the present invention.

### Example 1. Preparation of Serum-Free RPMI 8866 Cell-Conditioned Medium

The human B-lymphoblastoid cell line RPMI 8866 was maintained in RPMI 1640 medium containing 5% heat-inactivated fetal calf serum (FCS). For preparation of serum free conditioned medium, cells were washed and suspended (10⁶ cells/ml) in serum free RPMI 1640 medium containing 10⁻⁷ M phorbol-12-13-dibutyrrte (PdBU) and cultured for 48 hours at 37°C, 5% CO₂. The cell free supernatants were harvested by filtration through a 0.2 µm filter [Durapore® hydrophilic cartridge filter, Millipore, Bedford, MA], and Tween-20 and phenylmethylsulfonyl-fluoride (PMSF) were added to 0.02% and 0.1 mM, respectively. The cell conditioned medium was then concentrated 50 fold under pressure using an ultra-filtration cartridge [Spiral-Wound, S1, Amicon, Danvers, MA].

### Example 2. Purification of NKSF from Conditioned Medium

The following procedures are presently employed to obtain homogeneous NKSF protein from RPMI 8866 conditioned medium, as described in Example 1 above.

### a. Anion Exchange Cartridge Chromatography

Two liters of the crude concentrated conditioned medium were diluted with distilled water to a conductivity of 6m Os/cm and adjusted to pH 8 with 1 M Tris-HC1 buffer (pH 8). The concentrate was then applied to five QAE Zetaprep 250 cartridges [Pharmacia] connected in parallel and previously equilibrated with 0.1 M Tris-HC1 buffer (pH 8) at a flow rate of 150 ml/min. Unless otherwise cited, all the buffers used for purification contained 0.02% Tween-20 and 0.1 mM PMSF. The cartridges were washed with 3 liters of 0.1 M Tris-HC1 buffer (pH 6.8) followed by washing with 1.5 liters of 0.5 M NaCl in 0.1 M Tris-HC1 buffer (pH 6.8) and 300 ml fractions were collected. The NKSF activity was eluted with the 0.5 M NaCl-containing wash.

### b. Lentil-Lectin Sepharose Chromatography

NKSF-containing fractions from two separate QAE Zetaprep elutions were pooled and applied directly to a column (2.5x15 cm) of lentil-lectin Sepharose 4B [Pharmacia] which had been equilibrated with 20 mM Tris-HC1 buffer (pH 7.2). After washing with five column volumes of equilibration buffer, the column was eluted with three column volumes of 20 mM Tris-HCl buffer (pH 7.2) containing 0.2 M a-methyl-D-mannopyranoside [Sigma] and 0.5 M NaCl. Approximately half of the NKSF activity was bound by the column and was recovered in the fractions eluted with α-methyl-D-mannopyranoside.

### c. Hydroxylapatite Chromatography

Concentrated material from the pool of NKSF activity which bound to the lentil-lectin Sepharose column was dialyzed against 1 mM potassium phosphate buffer (pH 6.8) containing 0.1 mM CaCl₂ and 0.15 M NaCl and applied to a Biogel HT [BioRad] column (2x5 cm) previously equilibrated with 1 mM potassium phosphate buffer (pH 6.8) containing 0.1 mM CaCl₂. The column was washed with five column volumes of equilibration buffer and eluted with 100 ml of a linear gradient from 1 mM to 400 mM potassium phosphate buffer (pH 6.8) containing 0.15 M NaCl. 4 ml fractions were collected and tested for NKSF activity. A single peak of activity emerged from the column between the approximately 200 mM and 300 mM potassium phosphate.

### d. Heparin Sepharose Chromatography

Eluted NKSF-containing fractions from the Biogel HT column were pooled and dialyzed against 20 mM sodium phosphate buffer (pH 7.2) and applied to a Heparin Sepharose [Pierce, Rockford, IL] column (1x10 cm). The column was washed with five column volumes of 20 mM sodium phosphate buffer (pH 7.2) and eluted with the same buffer containing 1 M NaCl. 3 ml fractions were collected and NKSF activity measured. Essentially all of the activity was bound by the Heparin column and recovered in the 1 M NaCl wash.

### e. Mono Q Chromatography

Pooled fractions from the Heparin Sepharose column were dialyzed against 20 mM Tris-HC1 buffer (pH 6.8) containing 1% ethylene glycol and 0.1 mM PMSF but no Tween-20 (buffer A) and concentrated to 2 ml using a stirred cell [Amicon] with a YM 10 membrane. The sample was applied to a Mono Q (5/5) column [Pharmacia-FPLC apparatus] and eluted with a linear gradient from 0 M to 1 M NaCl in buffer A (pH 6.8). 0.5 ml fractions were collected and tested for NKSF activity. The activity emerged from the column as a single peak between approximately 220 mM and 270 mM NaCl.

### f. Gel Filtration Chromatography

Pooled fractions containing NKSF activity from the Mono Q column were concentrated to 100 microliters by Speedvac Concentrator [Savant, Farmingdale, NY] and applied to a FPLC Superose 12 column. Chromatography was run with 50 mM sodium phosphate buffer (pH 7.2) containing 0.15 M NaCl, 1% ethylene glycol and 0.1 mM PMSF. Flow rate was 0.6 ml/minute and 0.5 ml fractions were collected. NKSF protein (70 kd) was separated from the approximately 37 kd protein contaminant.

Alternatively, the pooled Mono-Q fractions may be subjected to reverse-phase HPLC (C8 column) prior to the step (f) described above, to separate the protein contaminant from the active 70 kd protein.

### Example 3. Sodium Dodecvl Sulfate-Polyacrylamide Gel Electrophoresis

SDS-PAGE was performed according to the method of Laemmli [Laemmli, U. K., Nature, 227:680-685 (1970)] on 10% acrylamide slab gels (0.75 mm thickness). After electrophoresis the gels were either stained by the silver-nitrate method using a silver staining reagents [BioRad] or cut into 2 mm slices and eluted in 0.5 ml RPMI medium for 4 hours at 24°C and assayed for NKSF activity. Apparent molecular weight was determined with protein standards, phospholipase b (94 kd), bovine serum albumin (67 kd), ovalbumin (43 kd), carbonic anhydrase (30 kd), soybean trypsin inhibitor (20 kd) and lactalbumin (14.4 kd).

SDS-PAGE analysis (non-reducing conditions) of the Mono Q column fractions (Example 2, step (e)) beginning with several fractions which eluted before the NKSF activity, continuing right through the active fractions and ending with fractions which eluted after the peak of NKSF activity, revealed that the presence of two proteins (70 kd and 37 kd) correlated with the presence of NKSF activity in the various Mono Q fractions. The active fractions were rerun on a second non-reducing gel and the proteins were eluted from the regions corresponding to the 70 kd and 37 kd bands and tested for NKSF activity. The activity all correlated with the 70 kd species indicating that this protein is NKSF.

The 70 kd species was eluted from the gel, iodinated using chloramine T [Sigma, St. Louis, MO] and rerun on a second SDS gel after boiling for two minutes in the presence of the reducing agent, β-mercaptoethanol (10%). Under these conditions, the 70 kd species resolved into two distinct subunits of molecular weights 40kd and 30kd, indicating that the native NKSF may be a disulfide-bonded heterodimer of these subunit polypeptides. Alternatively, NKSF may be a dimer formed by multiples of the larger or smaller subunits. The reduction of the native 70kd NKSF appeared to destroy all of its ability to induce peripheral blood lymphocyte production of gamma interferon.

### Example 4. Recovery of Protein

Starting with 500 liters of RPMI 8866 cell-free conditioned medium, the final pooled active fractions from the Mono Q column contained approximately 10 µg of protein, estimated from the intensities of silver staining as compared to control proteins analyzed in parallel on the same gel. Approximately 6 µg of this corresponded to the 70 kd NKSF protein. The estimated specific activity of the 70 kd NKSF is 1x10⁷ u/mg. The overall recovery of NKSF activity in the preparation was 2%.

### Example 5. NKSF Protein Composition

Homogeneous NKSF is reduced as described in the SDS-PAGE example above and digested with trypsin. Alternatively, non-reduced NKSF may be obtained from a reverse-phase HPLC column and digested with trypsin. Nine tryptic fragments are isolated having the following amino acid sequences with tentatively identified amino acids in parentheses:

Fragment 1 - L-T-I-Q-V
Fragment 2 - L-M-D-P-K
Fragment 3 - K-Y-E-N-Y-T
Fragment 4 - I-W-E-L-K
Fragment 5 - V-M-S-Y-L-N-A
Fragment 6 - (A)-V-S-N-M-L-Q-K
Fragment 7 - N-A-S-I-S-V
Fragment 8 - T-F-L-R
Fragment 9 - D-I-I-K-P-D-P-P-K.

Additionally, the amino acid sequences of the amino termini of each subunit of NKSF were determined from the isolated 40 kd and 30kd species of NKSF after reduction, as described in Example 3. The amino terminal sequence from the 40kd subunit was as follows: I-W-E-L-K-K-D-V-Y-V-V-E-L-D-W-Y-P-D-A-P-G-E-M. The amino terminal sequence above as well as Fragments 1, 3, 4, 8 and 9 proved to be derived from the amino acid sequence of the clone of the larger subunit identified in Table I above.

The amino terminal sequence from the 30kd smaller subunit was as follows, with tentatively identified amino acids enclosed in parentheses: (X)-N-L-P-V-A-(P)-P-D-P-(S)-M-F-P. The (X) indicates that the first residue in this sequence could not be determined.

Probes consisting of pools of oligonucleotides or unique oligonucleotides are designed according to the method of R. Lathe, J. Mol. Biol., 183(1):1-12 (1985). The oligonucleotide probes are synthesized on an automated DNA synthesizer.

Because the genetic code is degenerate (more than one codon can code for the same amino acid) a mixture of oligonucleotides must be synthesized that contains all possible nucleotide sequences encoding the amino acid sequence of the tryptic fragment. It may be possible in some cases to reduce the number of oligonucleotides in the probe mixture based on codon usage because some codons are rarely used in eukaryotic genes, and because of the relative infrequency of the dinucleotide CpG in eukaryotic coding sequences [see J. J. Toole et al, Nature, 312:342-347 (1984)]. The regions of the amino acid sequences used for probe design are chosen by avoiding highly degenerate codons where possible. The oligonucleotides are synthesized on an automated DNA synthesizer and the probes are then radioactively labelled with polynucleotide kinase and ³²P-ATP.

cDNA is then synthesized from polyadenylated RNA from the RPMI 8866 cell line and cloned into lambda ZAP [Stratagene Cloning Systems, La Jolla, CA] or other suitable vectors using established techniques (see Toole et al cited above). Recombinants from this library are plated and duplicate nitrocellulose replicas made of the plates. The oligonucleotides are kinased with ³²P gamma ATP and hybridized to the replicas at a temperature predicted from the length and base composition of the probes [See, J. Singer-Sam et al, Proc. Nat'l. Acad. Sci. USA, 80;802-806 (1983) and S.V. Suggs et al, in "Developmental Biology Using Purified Genes", ICN-UCLA Symposium on Molecular and Cellular Biology, eds. Brown D.D. and Fox, C.F. (Academic, NY), vol. 23, pp. 683-693 (1981)] in standard hybridization solution overnight. The filters are then washed in 0.5XSSC at the same temperature until the background radioactivity is lowered to an acceptable level to allow autoradiography. Alternatively, the hybridization and washes may be performed in the presence of tetraalkylammonium salt solution [See K. A. Jacobs et al, Nucl. Acids Res., 16:4637-4650 (1988).] Duplicate positives are plaque purified. Clones containing all or a portion of the nucleotide sequence necessary to encode human NKSF are obtained. One of the clones obtained according to this procedure is described above and deposited as ATCC.

### Example 6. Expression of Recombinant Human NKSF

To produce NKSF, the cDNAs encoding its subunits are transferred into appropriate expression vectors, of which numerous types are known in the art for mammalian, insect, yeast, fungal and bacterial expression, by standard molecular biology techniques. One such vector for mammalian cells is pXM [Y. C. Yang et al, Cell, 47:3-10 (1986)]. This vector contains the SV40 origin of replication and enhancer, the adenovirus major late promoter, a cDNA copy of the adenovirus tripartite leader sequence, a small hybrid intervening sequence, an SV40 polyadenylation signal and the adenovirus VA I gene, in appropriate relationships to direct the high level expression of the desired cDNA in mammalian cells [See, e.g., Kaufman, Proc. Natl. Acad. Sci. USA, 82:689-693 (1985)]. The pXM vector is linearized with the endonuclease enzyme XhoI and subsequently ligated in equimolar amount separately to the cDNA encoding the NKSF subunits that were previously modified by addition of synthetic oligonucleotides [Collaborative Research, Lexington, MA] that generate Xho I complementary ends to generate constructs for expression of each subunit of NKSF. If the two polypeptides derive from two different mRNAs, two different cDNAs must be expressed simultaneously in the same host or independently in different hosts and the subunits purified separately. The final active NKSF is assembled by renaturation of the individual subunits.

If two subunits of NKSF are derived from a single mRNA, i.e., they are generated by proteolytic cleavage of a single precursor polypeptide, the vector is ligated in equimolar amount to the individual cDNA in a similar manner as described above. The corresponding cDNA can be expressed in various hosts with appropriate vectors.

### a. Mammalian Cell Expression

To obtain expression of the NKSF protein for use in the assays described below, the pXM constructs containing the cDNAs for the individual subunits (or the single cDNA encoding both subunits if they are derived from a single precursor) are mixed and transfected into COS cells, for example. The conditioned medium from the transfected COS cells contains NKSF biological activity as measured in the gamma interferon induction assay.

The mammalian cell expression vectors described herein may be synthesized by techniques well known to those skilled in this art. The components of the vectors, e.g. replicons, selection genes, enhancers, promoters, and the like, may be obtained from natural sources or synthesized by known procedures. See, Kaufman et al, J. Mol. Biol., 159:511-521 (1982); and Kaufman, Proc. Natl. Acad. Sci., USA, 82:689-693 (1985). Exemplary mammalian host cells include in particular primate cell lines and rodent cell lines, including transformed cell lines. Normal diploid cells, cell strains derived from in vitro culture of primary tissue, as well as primary explants, are also suitable. Candidate cells need not be genotypically deficient in the selection gene so long as the selection gene is dominantly acting. For stable integration of the vector DNAs, and for subsequent amplification of the integrated vector DNAs, both by conventional methods, CHO cells may be employed. Alternatively, the vector DNA may include all or part of the bovine papilloma virus genome [Lusky et al, Cell, 36:391-401 (1984)] and be carried in cell lines such as C127 mouse cells as a stable episomal element. Other suitable mammalian cell lines include but are not limited to, HeLa, COS-1 monkey cells, mouse L-929 cells, 3T3 lines derived from Swiss, Balb-c or NIH mice, BHK or HaK hamster cell lines.

Because the NKSF protein is a dimer, the two subunits, if different, e.g. a larger and a smaller subunit, must be expressed simultaneously in the same host or separately and refolded together to yield active NKSF. If, however, the NKSF subunits are generated by cleavage of a precursor encoded by a single cDNA, then, at least in mammalian cells containing the appropriate protease, the single cDNA can be expressed to yield functional NKSF.

Where the two subunits require simultaneous expression in mammalian cells, the two cDNAs must be introduced into the cells using two different selectable genes or markers. As discussed below in Example 7, this can readily be achieved in CHO cells using the dihydrofolate reductase (DHFR) gene as one marker and adenosine deaminase (ADA) as the other marker. Any combination of two genes which can be independently selected in any mammalian cell line are useful for this purpose. For example, a CHO cell line is independently developed for expression of one subunit under ADA selection and a different cell line is developed for expression of the other subunit under DHFR selection. The cell lines are fused in polyethylene glycol under double selection to yield stable lines expressing both subunits. Alternatively, the DNAs are introduced simultaneously or sequentially into the same cells, thereby yielding lines expressing active NKSF.

Stable transformants are then screened for expression of the product by standard immunological, biological or enzymatic assays. The presence of the DNA and mRNA encoding the NKSF polypeptides may be detected by standard procedures such as Southern blotting and RNA blotting. Transient expression of the DNA encoding the polypeptides during the several days after introduction of the expression vector DNA into suitable host cells, such as COS-1 monkey cells, is measured without selection by activity or immunological assay of the proteins in the culture medium.

One skilled in the art can also construct other mammalian expression vectors comparable to the pXM vector by, e.g., inserting the DNA sequences of the NKSF subunits into the respective plasmids using appropriate enzymes and employing well-known recombinant genetic engineering techniques and other known vectors, such as pJL3 and pJL4 [Gough et al., EMBO J., 4:645-653 (1985)]] and pMT2 (starting with pMT2-VWF, ATCC #67122; see PCT application PCT/US87/00033). The transformation of these vectors with both NKSF subunits into appropriate host cells can result in expression of the NKSF polypeptides.

### b. Bacterial Expression Systems

Similarly, one skilled in the art could manipulate the sequences encoding the NKSF subunits by eliminating any mammalian regulatory sequences flanking the coding sequences and inserting bacterial regulatory sequences to create bacterial vectors for intracellular or extracellular expression of the NKSF subunits of the invention by bacterial cells. The DNA encoding the NKSF polypeptides may be further modified to contain different codons to optimize bacterial expression as is known in the art. Preferably the sequences encoding the mature NKSF subunits are operatively linked in-frame to nucleotide sequences encoding a secretory leader polypeptide permitting bacterial expression, secretion and processing of the mature NKSF polypeptides, also by methods known in the art. The simultaneous expression of both subunits of NKSF in E. coli using such secretion systems is expected to result in the secretion of the active heterodimer. This approach has yielded active chimeric antibody fragments [See, e.g., Bitter et al, Science, 240:1041-1043 (1988)].

Alternatively, the individual subunits are expressed in the mature form separately from the two different cDNAs in E. coli using vectors for intracellular expression and the subunits are isolated separately, mixed and refolded by procedures well known in the art. See, for example, U.S. Patent 4,512,922.

The compounds expressed through either route in bacterial host cells may then be recovered, purified, and/or characterized with respect to physicochemical, biochemical and/or clinical parameters, all by known methods.

### c. Insect or Yeast Cell Expression

Similar manipulations can be performed for the construction of an insect vector for expression of NKSF polypeptides in insect cells [See, e.g., procedures described in published European patent application 155,476]. If the NKSF subunits are derived from a single cDNA, this cDNA will be expressed in insect cells. Alternatively, if the NKSF subunits are derived from two different cDNAs, each subunit is separately inserted into an insect cell vector and the two resulting vectors co-introduced into insect cells to express biologically active NKSF.

Similarly yeast vectors are constructed employing yeast regulatory sequences to express in yeast cells either the individual NKSF subunits simultaneously, or, if the protein is derived from a single precursor, the cDNA encoding that precursor, to yield secreted extracellular active NKSF heterodimer. Alternatively the individual subunits may be expressed intracellularly in yeast, the individual polypeptides isolated and finally, refolded together to yield active NKSF. [See, e.g., procedures described in published PCT application WO 86/00639 and European patent application EP 123,289.]

### Example 7. Construction of CHO Cell Lines Expressing High Levels of NKSF

One method for producing high levels of the NKSF protein of the invention from mammalian cells involves the construction of cells containing multiple copies of the two cDNAs encoding the individual NKSF subunits or of the cDNA encoding the NKSF precursor if the subunits are derived from a single polypeptide.

In the latter case, the single cDNA is co-transfected with an amplifiable marker, e.g., the DHFR gene in the case of cells which are grown in medium containing increasing concentrations of methotrexate (MTX) according to the procedures of Kaufman and Sharp, J. Mol. Biol., (1982) supra. This approach can be employed with a number of different cell types.

For example, the pXM vector containing the NKSF precursor gene in operative association with other plasmid sequences enabling expression thereof (Example 6) is introduced into DHFR-deficient CHO cells, DUKX-BII, along with a DHFR expression plasmid such as pAdD26SVpA3 [Kaufman, Proc. Natl. Acad. Sci. USA, 82:689-693 (1985)] by calcium phosphate coprecipitation and transfection. DHFR expressing transformants are selected for growth in alpha media with dialyzed fetal calf serum. Transformants are checked for expression of NKSF by bioassay, immunoassay or RNA blotting and positive pools are subsequently selected for amplification by growth in increasing concentrations of MTX (sequential steps in 0.02, 0.2, 1.0 and 5uM MTX) as described in Kaufman et al., Mol. Cell Biol., 5:1750 (1983). The amplified lines are cloned, and NKSF protein expression is monitored by the gamma interferon induction assay. NKSF expression is expected to increase with increasing levels of MTX resistance.

If the two NKSF polypeptides are each derived from separate mRNAs, each corresponding cDNA is expressed simultaneously in CHO cells. Two different selectable markers, e.g., DHFR and ADA, may be employed. One of the cDNAs is expressed using the DHFR system using, e.g., the vector pXM to express one of the NKSF subunits and pAdD26SVpA3 to express DHFR as described above for a single precursor NKSF protein. The second subunit is also expressed using the vector pXM but the marker is obtained through co-transfection with the plasmid pSV2ADA [Kaufman et al, Proc. Natl. Acad. Sci. USA, 83:3136 (1986)], which directs expression of ADA in mammalian cells. The pXM vector construct containing the second subunit is transfected into DHFR-deficient CHO DUKX-BII cells along with pSV2ADA. The transfected cells are selected for growth in increasing concentrations of 2'-deoxycoformycin (dCF) beginning with 0.01 micromolar with subsequent step-wise increments up to 40 micromolar. The expression of the individual cDNAs (one subunit under DHFR selection in one cell line and the other subunit under ADA selection in a second cell line) is monitored through a combination of mRNA blotting to test for transcription and immunoanalysis to test for protein production. The cells which express one of the subunits under ADA selection and the cells which express the other subunit under DHFR selection are finally fused in polyethylene glycol using methods well established in the art to yield a single cell line, resistant to both dCF and MTX and expressing both subunits to yield biologically active NKSF.

A cell line expressing either subunit under either drug selection may be generated. Secondarily the cDNA expressing the other subunit may be introduced under the second drug selection to yield cells expressing both subunits simultaneously. (See, e.g., published PCT International Application WO88/08035 for an exemplary description of independently amplifying a first gene linked to a DHFR gene and a second gene linked to an ADA gene.)

Additionally the two pXM constructs expressing both NKSF subunits may be mixed with the plasmid for expression of DHFR and the plasmid expressing ADA. The combined selection by both drugs may be used and transformants tested for NKSF activity directly to obtain cell lines expressing the heterodimer.

In any of the expression systems described above, the resulting cell lines can be further amplified by appropriate drug selection, resulting cell lines recloned and the level of expression assessed using the gamma interferon induction assay described herein.

### Example 8. Biological Activities of Human NKSF

The following assays were performed using either the homogeneous NKSF described in Example 2 or a partially purified version of NKSF. The recombinant version of the molecule is expected to exhibit NKSF biological properties in the same assays or other assays.

When fresh human peripheral blood mononuclear cells (PBMC) or phytohemagglutinin (PHA)-induced blasts are cultured with NKSF, significant amounts of gamma interferon are detected in the supernatant. Moreover, NKSF exhibits a synergistic effect with IL-2, phorbol dibutyrate (PdBu), and PHA in inducing gamma interferon production. Northern blot analyses show that NKSF, alone or in combination with other factors, induces accumulation of gamma interferon mRNA. Gamma interferon mRNA was found in both purified T and NK cell populations. Preincubation with the protein synthesis inhibitor, cycloheximide (CHX), leads to a superinduction of gamma interferon mRNA following stimulation with NKSF. HLA-DR(+) accessory cells are required for gamma interferon production by T and NK cells. Induction of gamma interferon mRNA can be detected as early as 1 hour after treatment with NKSF of PHA blasts. The details of the assay are described below.

### a. Gamma Interferon Induction Assay

NKSF activity was measured by the induction of gamma interferon (gamma-IFN) expression in cultures of human peripheral blood lymphocytes (PBLs). In the assay, 100 µl of human PBLs suspended (10⁷ cells/ml) in RPMI 1640 culture medium supplemented with 10% heat-inactivated FCS was added to 100 µl of sample to be tested in a microtiter plate [U-bottom, 96-well, Costar, Cambridge, MA] and incubated for 18 hours at 37°C, 5% CO₂. Samples to be tested included purified NKSF, dialyzed cell free supernatant from 48 hour phorbol diester stimulated RPMI 8866 cells, and recombinant IL-2 [Genetics Institute, Inc., PCT application WO85/05124]. After incubation, 100 µl of cell free supernatant was withdrawn from each well and the level of gamma-IFN produced measured by radioimmunoassay [Centocor Gamma Interferon Radioimmunoassay, Centocor, Malvern, PA]. One unit of NKSF per ml is the concentration required to produce one-half of the maximal gamma-IFN produced in the presence of optimal concentrations of NKSF.

There was a linear positive correlation between the amount of gamma-IFN produced in each well and the amount of NKSF in culture.

In addition to gamma-IFN, NKSF induces T and NK cells to produce GM-CSF and tumor necrosis factor. The assay of production of these cytokines is performed as above and the supernatant is assayed for the presence of the cytokines by specific biological assays or by radioimmunoassays [Cuturi et al, J. Exp. Med., 165:1581-1594 (1987)]. Alternatively, the induction of the cytokine genes is measured by evaluating the accumulation of mRNA transcripts of the three cytokines in the lymphocytes treated with NKSF. Lymphocytes are cultured for 4 to 18 hours with NKSF, RNA is extracted by established methods, fractionated by agarose gel electrophoresis, blotted on nitrocellulose, and hybridized with ³²P-labeled cDNA probes for the IFN-gamma, GM-CSF, or tumor necrosis factor genes (Northern blotting). Extent of hybridization is determined by autoradiography and densitometry.

NKSF induces production of IFN-gamma and TNF from purified human NK cells. When assayed as described under the gamma interferon induction assay of part (a) above, NK cells are able to lyse various target cells by two mechanisms. One mechanism is spontaneous lysis, in the absence of specific sensitization, of a variety of target cells, including leukemia- and solid tumor-derived cell lines, virus-infected cells, and, in some cases, normal cell lines. The second mechanism is ADCC. Preliminary evidence indicates that NKSF may enhance the ability of NK cells to lyse more efficiently target cells coated with IgG antibodies with an Fc portion able to bind to the NK cell Fc receptor.

### b. NK Assay

In order to assay for the enhancement of NK cell spontaneous cytotoxicity by NKSF, PBLs or purified NK cells (5x10⁶ cells/ml) are incubated for 18 hours in RPMI 1640 medium, containing 10% heat inactivated FCS, in the presence of various dilutions of NKSF. PBLs are then washed and added, at PBL-target cells ratios from 1:1 to 100:1, to 10^{4 51}Cr-labeled target cells in a U-bottomed microtiter plate (final volume 200 µl). After 4 hours, the plates are centrifuged, the cell-free supernatant is collected and lysis of target cells is evaluated by the release of the ⁵¹Cr-label from the cells. NKSF increases several-fold the cytotoxicity of NK cells when assayed against the following target cells: malignant hematopoietic cell lines (i.e. K562, Daudi, U937, HL-60, ML3, Molt 4, Jurkat, THP-1), solid tumor-derived cell line (rhabdomyosarcoma, melanoma), and normal foreskin-derived fibroblast strains. The enhancement of NK cell-mediated cytotoxicity by NKSF is not secondary to the production of IFN-gamma, tumor necrosis factor, or IL-2, produced by the PBL treated with NKSF. The cytotoxic assay, the methods for NK cell purification, and for the quantitative evaluation of enhancement of NK cell-mediated enhancement by cytokines are described in detail in G. Trinchieri et al, J. Exp. Med., 147:1314 (1978); G. Trinchieri et al, J. Exp. Med., 160:1147 (1984); and B. Perussia et al, Natural Immunity and Cell Growth Regulation, 6:171-188 (1987).

### c. ADCC Assay

In a standard antibody dependent cell mediated cytotoxity assay, preliminary results show that partially purified NKSF of the present invention enhances NK cell killing of antibody coated tumor target cells in a dose dependent manner. For antibodies capable of binding to the Fc receptor of the NK cell, the ADCC response of NK cells was enhanced by the addition of NKSF.

### d. Co-mitogenic effect of NKSF

PBLs (0.5x10⁶/ml) are cultured in 200 µl of RPMI 1640 medium supplemented with 10% heat inactivated human AB serum. After 3 and 6 days the PBLs are pulsed for 6 hours with ³H-thymidine and DNA synthesis (proliferation) is evaluated by the ³H-thymidine uptake in the cells by collecting the cells on glass filters using a Skatron cell harvester and counting the cell-associated ³H-Thymidine by liquid scintillation using a Packard Tricarb beta counter. NKSF has minimal effect on PBL proliferation by itself, but is strongly co-mitogenic with phytohemagglutinin (PHA-M Welcome, 1:100) at day 6 of culture and with phorbol diesters (TPA or PDBu, 10⁻⁸ or 10⁻⁷M, respectively) at both day 3 and day 6. Cell cycle analysis is performed by flow cytofluorometry (Cytofluorograf 50H, Ortho Diagnostics) using a technique combining DNA staining with immunofluorescence staining according to London et al, J. Immunol., 137:3845 (1986). This analysis has shown that the PBLs affected by the co-mitogenic effect of NKSF are T cells either CD4 or CD8 positive.

### e. GM-CSF Induction Assay

Induction of GM-CSF expression in cultures of human PBLs was measured. In the assay, 100 µl of human PBLs suspended (10⁷ cells/ml) in RPMI 1640 culture medium supplemented with 10% heat-inactivated FCS was added to 100 µl of sample to be tested in a microtiter plate [U-bottom, 96-well, Costar, Cambridge, MA] and incubated for 18 hours at 37°C, 5% CO₂. After incubation, 100 µl of cell-free supernatant was withdrawn from each well and the level of GM-CSF produced measured by enzyme-linked immunosorbent assay (ELISA) using two murine monoclonal antibodies against human GM-CSF (3/8.20.5 and 2/3.1, supplied by Genetics Institute, Inc.) recognizing different epitopes. Using recombinant human GM-CSF (Genetics Institute, Inc.) as a standard, the detection limit of this assay was 50 pg/ml.

Numerous modifications and variations in practice of this invention are expected to occur to those skilled in the art.

## Claims

1. Natural killer cell stimulating factor protein (NKSF) comprising a subunit encoded by a DNA sequence comprising a DNA sequence selected from
(a) the DNA sequence shown in Table I;
(b) DNA sequences which hybridize to the DNA sequence of (a);
(c) allelic variations, analogues, derivatives or fragments of the DNA sequence of (a) or (b); or
(d) DNA sequences which differ in codon sequence due to the degeneracy of the genetic code;
wherein the NKSF protein containing said subunit retains NKSF activity and is active in a gamma interferon induction assay.

2. The protein according to claim 1 wherein said subunit has
(a) an apparent molecular weight of approximately 40 kd on SDS-PAGE under reducing conditions; and
(b) the following N-terminal amino acid sequence I-W-E-L-K-K-D-V-Y-V-V-E-L-D-W-Y-P-D-A-P-G-E-M.

3. The protein according to claim 2 wherein said subunit has the following N-terminal amino acid sequence 
M-C-H-Q-Q-L-V-I-S-W-

F-S-L-V-F-L-A-S-P-L-V-A-I-W-E-L-K-K-D-V-Y-V-V-E-L-D-W-Y-

P-D-A-P-G-E-M-V-V-L-T-C-D-T-P-E-E-D-G-I-T-W-T-L-D-Q-S-S-

E-V-L-G-S-G-K-T-L-T-I-Q-V-K-E-F-G-D-A-G-Q-Y-T-C-H-K-G-G-

E-V-L-S-H-S-L-L-L-L-H-K-K-E-D-G-I-W-S-T-D-I-L-K-D-Q-K-E-

P-K-N-K-T-F-L-R-C-E-A-K-N-Y-S-G-R-F-T-C-W-W-L-T-T-I-S-T-

D-L-T-F-S-V-K-S-S-R-G-S-S-D-P-Q-G-V-T-C-G-A-A-T-L-S-A-E-
 
R-V-R-G-D-N-K-E-Y-E-Y-S-V-E-C-Q-E-D-S-A-C-P-A-A-E-E-S-L-

P-I-E-V-M-V-D-A-V-H-K-L-K-Y-E-N-Y-T-S-S-F-F-I-R-D-I-I-K-

P-D-P-P-K-N-L-Q-L-K-P-L-K-N-S-R-Q-V-E-V-S-W-E-Y-P-D-T-W-

S-T-P-H-S-Y-F-S-L-T-F-C-V-Q-V-Q-G-K-S-K-R-E-N-S-R-V-D-G-

I,
 or fragments thereof.

4. The protein according to claim 1 comprising a subunit having
(a) an apparent molecular weight of approximately 30 to 35 kd on SDS-PAGE under reducing conditions; and
(b) the N-terminal amino acid sequence X-N-L-P-V-A-(P or X)-P-D-P-(S or X or T)-M-F-P, wherein X is any amino acid.

5. The protein according to any one of claims 1 to 4 characterized by comprising one or more of the following amino acid sequences: 
L-T-I-Q-V;
K-Y-E-N-Y-T;
L-M-D-P-K;
I-W-E-L-K;
V-M-S-Y-L-N-A;
(A)-V-S-N-M-L-Q-K;
N-A-S-I-S-V;
T-F-L-R;
D-I-I-K-P-D-P-P-K;
I-W-E-L-K-K-D-V-Y-V-V-E-L-D-W-Y-P-D-A-P-G-E-M; and
X-N-L-P-V-A-(P or X)-P-D-P-(S or X or T)-M-F-P,
 wherein X is any amino acid.

6. The protein according to any one of claims 1 to 5 having one or more of the following characteristics:
(1) an apparent molecular weight under non-reducing conditions on SDS-PAGE of approximately 70 to 80 kd;
(2) a subunit having an apparent molecular weight under reducing conditions on SDS-PAGE of approximately 40 kd;
(3) a subunit having an apparent molecular weight under reducing conditions on SDS-PAGE of approximately 30 to 35 kd;
(4) an isoelectric point of 3.4 or 4.8 on an isoelectric focusing gel;
(5) elution from hydroxylapatite column as a single peak;
(6) elution from heparin-sepharose column as a single peak;
(7) elution from an FPLC Mono-Q® column as a single peak;
(8) biological activity in a gamma IFN inducing assay with PBLs;
(9) biological activity in a GM-CSF inducing assay with PBLs;
(10) biological activity in activating NK cells to kill leukemia and tumor-derived cells;
(11) biological activity in a tumor necrosis factor induction assay using PHA-activated T lymphocytes;
(12) co-mitogenic activity on peripheral blood T lymphocytes.

7. The protein according to any one of claims 1 to 6 produced by subjecting conditioned medium from RPMI 8866 to sequential purification through a QAE Zeta Prep® cartridge, a lentil lectin column, a hydroxylapatite column, a heparin sepharose column and a fast protein liquid chromatography Mono-Q® column, wherein said protein elutes from the latter column as a single peak.

8. The protein according to any one of claims 1 to 6 produced by culturing a cell line transformed with a DNA sequence encoding said protein, said DNA sequence being in operative association with an expression control sequence therefor.

9. The protein according to any one of claims 1 to 8 having a specific activity in a gamma interferon induction assay of greater than 1x10⁷ dilution units/mg.

10. A subunit of a protein according to any one of claims 1 to 9.

11. A process for preparing a homogeneous protein according to any one of claims 1 to 7 comprising subjecting conditioned medium from RPMI 8866 to sequential purification through a QAE Zeta Prep® cartridge, a lentil lectin column, a hydroxylapatite column, a heparin sepharose column and a fast protein liquid chromatography Mono-Q® column, wherein said protein elutes from the latter column as a single peak.

12. The process according to claim 11 further comprising subjecting the Mono-Q® column eluate to gel filtration chromatography.

13. The process according to claim 11 or 12 optionally including a reverse phase HPLC purification before said gel filtration chromatography.

14. A DNA sequence coding for NKSF or a subunit thereof, comprising a DNA sequence selected from
(a) the DNA sequence 
GAATTCCGTCGACTCTAGAGGCCCAGAGCAAGATGTGTCACCAGCAGTTGGTCATCTCTT

80 100 120

GGTTTTCCCTGGTTTTTCTGGCATCTCCCCTCGTGGCCATATGGAACTGAAGAAAGATG
 
140 180
TTTATGTCGTAGAATTGGATTGGTATCCGGAGGCCCCTGGAGAAATGGTGGTCCTCACCT
240
GTGACACCCCTGAAGAAGATGGTATCACCTGGACCTTGGACCAGAGCAGTGAGGTCTTAG
300
GCTCTGGCAAAACCCTGACCATCCAAGTCAAAGAGTTTGGAGATGCTGGCCAGTACACCT
360
GTCACAAAGGAGGCGAGGTTCTAAGCCATTCGCTCCTGCTGCTTCACAAAAAGGAAGATG
420
GAATTTGGTCCACTGATATTTTAAAGGACCAGAAAGAACCCAAAAATAAGACCTTTCTAA
480
GATGCGAGGCCAAGAATTATTCTGGACGTTTCACCTGCTGGTGGCTGACGACAATCAGTA
540
CTGATTTGACATTCAGTGTCAAAAGCAGCAGAGGCTCTTCTGACCCCCAAGGGGTGACGT
600
GCGGAGCTGCTACACTCTCTGCAGAGAGAGTCAGAGGGGACAACAAGGAGTATGAGTACT
660
CAGTGGAGTGCCAGGAGGACAGTGCCTGCCCAGCTGCTGAGGAGAGTCTGCCCATTGAGG
720
TCATGGTGGATGCCGTTCACAAGCTCAAGTATGAAAACTACACCAGCAGCTTCTTCATCA
780
GGGACATCATCAAACCTGACCCACCCAAGAACTTGCAGCTGAAGCCATTAAAGAATTCTC
840
GGCAGGTGGAGGTCAGCTGGGAGTACCCTGACACCTGGAGTACTCCACATTCCTACTTCT
900
CCCTGACATTCTGCGTTCAGGTCCAGGGCAAGAGCAAGAGAGAAAACTCTAGAGTCGACG

GAATTC
(b) DNA sequences which hybridize to the DNA sequence of (a);
(c) allelic variations, analogues, derivatives or fragments of the DNA sequences of (a) or (b); or
(d) DNA sequences which differ in codon sequence due to the degeneracy of the genetic code
wherein said NKSF or a protein containing said subunit display NKSF activity and are active in a gamma interferon induction assay.

15. The DNA sequence according to claim 14 comprising a sequence of nucleotides encoding one or more of the following amino acid sequences: 
L-T-I-Q-V;
K-Y-E-N-Y-T;
L-M-D-P-K;
I-W-E-L-K;
V-M-S-Y-L-N-A;
(A)-V-S-N-M-L-Q-K;
N-A-S-I-S-V;
T-F-L-R;
D-I-I-K-P-D-P-P-K;
I-W-E-L-K-K-D-V-Y-V-V-E-L-D-W-Y-P-D-A-P-G-E-M; and
X-N-L-P-V-A-(P or X)-P-D-P-(S or X or T)-M-F-P;
 wherein X is any amino acid.

16. The DNA sequence according to claim 14 comprising a sequence of nucleotides encoding the amino acid sequence

M-C-H-Q-Q-L-V-I-S-W-F-S-L-V-F-L-A-S-P-L-V-A-I-
W-E-L-K-K-D-V-Y-V-V-E-L-D-W-Y-P-D-A-P-G-E-M-V-V-L-T-C-D-
T-P-E-E-D-G-I-T-W-T-L-D-Q-S-S-E-V-L-G-S-G-K-T-L-T-I-Q-V-
K-E-F-G-D-A-G-Q-Y-T-C-H-K-G-G-E-V-L-S-H-S-L-L-L-H-K-K-
E-D-G-I-W-S-T-D-I-L-K-D-Q-K-E-P-K-N-K-T-F-L-R-C-E-A-K-N-

Y-S-G-R-F-T-C-W-W-L-T-T-I-S-T-D-L-T-F-S-V-K-S-S-R-G-S-S-
D-P-Q-G-V-T-C-G-A-A-T-L-S-A-E-R-V-R-G-D-N-K-E-Y-E-Y-S-V-
E-C-Q-E-D-S-A-C-P-A-A-E-E-S-L-P-I-E-V-M-V-D-A-V-H-K-L-K-
Y-E-N-Y-T-S-S-F-F-I-R-D-I-I-K-P-D-P-P-K-N-L-Q-L-K-P-L-K-
N-S-R-Q-V-E-V-S-W-E-Y-P-D-T-W-S-T-P-H-S-Y-F-S-L-T-F-C-V-
Q-V-Q-G-K-S-K-R-E-N-S-R-V-D-G-I.

17. A vector comprising a DNA sequence of any one of claims 14 to 16.

18. The vector of claim 17 wherein said DNA sequence is operatively linked to (an) expression control sequence(s).

19. A cell transformed with the vector of claim 17 or 18.

20. The cell according to claim 19 which is a mammalian or bacterial cell.

21. A process for producing a protein according to any one of claims 1 to 6 or a subunit thereof according to claim 10 comprising culturing a transformed cell according to claim 19 or 20.

22. A pharmaceutical composition comprising a protein according to any one of claims 1 to 9 or a subunit thereof according to claim 10, in a pharmaceutically effective vehicle.

23. The composition according to claim 22 further comprising an additional cytokine, hematopoietin, growth factor or antibody capable of binding to the Fc portion of NK cells.

24. The composition according to claim 23 wherein said cytokine is IL-1, IL-2 or IL-6.

## Patentansprüche

1. Natürliches Killerzellen-Stimulationsfaktor-Protein (NKSF), umfassend eine Untereinheit, die durch eine DNA-Sequenz codiert wird, die eine DNA-Sequenz umfaßt, die ausgewählt ist unter
(a) der in Tabelle I gezeigten DNA-Sequenz;
(b) den mit der DNA-Sequenz unter (a) hybridisierenden DNA-Sequenzen;
(c) allelischen Variationen, Analogen, Derivaten oder Fragmenten der unter (a) oder (b) genannten DNA-Sequenz; oder
(d) DNA-Sequenzen, die sich wegen der Degeneriertheit des genetischen Codes in der Codonsequenz unterscheiden;
wobei das die besagte Untereinheit enthaltende NKSF-Protein die NKSF-Aktivität beibehält und in einem gamma-Interferon-Induktionstest aktiv ist.

2. Protein nach Anspruch 1, wobei die Untereinheit
(a) in einer SDS-PAGE unter reduzierenden Bedingungen ein apparentes Molekulargewicht von ungefähr 40 kD; und
(b) die nachfolgende N-terminale Aminosäuresequenz I-W-E-L-K-K-D-V-Y-V-V-E-L-D-W-Y-P-D-A-P-G-E-M
aufweist.

3. Protein nach Anspruch 2, wobei die Untereinheit die nachfolgende N-terminale Aminosäuresequenz M-C-H-Q-Q-L-V-I- 
M-C-H-Q-Q-L-V-I-
S-W-F-S-L-V-F-L-A-S-P-L-V-A-I-W-E-L-K-K-D-V-Y-V-V-E-L-D-W-Y-
P-D-A-P-G-E-M-V-V-L-T-C-D-T-P-E-E-D-G-I-T-W-T-L-D-Q-S-S-E-V-
L-G-S-G-K-T-L-T-I-Q-V-K-E-F-G-D-A-G-Q-Y-T-C-H-K-G-G-E-V-L-S-
H-S-L-L-L-L-H-K-K-E-D-G-I-W-S-T-D-I-L-K-D-Q-K-E-P-K-N-K-T-F-
L-R-C-E-A-K-N-Y-S-G-R-F-T-C-W-W-L-T-T-I-S-T-D-L-T-F-S-V-K-S-
S-R-G-S-S-T-P-Q-G-V-T-C-G-A-A-T-L-S-A-E-R-V-R-G-D-N-K-E-Y-E-
Y-S-V-E-C-Q-E-D-S-A-C-P-A-A-E-E-S-L-P-I-E-V-N-V-D-A-V-H-K-L-
K-Y-E-N-Y-T-S-S-F-F-I-R-D-I-I-K-P-D-P-P-K-N-L-Q-L-K-P-L-K-N-
S-R-Q-V-E-V-S-W-E-Y-P-D-T-W-S-T-P-H-S-Y-F-S-L-T-F-C-V-Q-V-Q-
G-K-S-K-R-E-N-S-R-V-D-G-I
 oder Fragmente derselben aufweist.

4. Protein nach Anspruch 1, umfassend eine Untereinheit, die
(a) in der SDS-PAGE unter reduzierenden Bedingungen ein apparentes Molekulargewicht von ungefähr 30 bis 35 kD; und
(b) die N-terminale Aminosäuresequenz X-N-L-P-V-A-(P oder X)-P-D-P-(S oder X oder T)-M-F-P, in der X eine beliebige Aminosäure darstellt,
aufweist.

5. Protein nach einem der Ansprüche 1 bis 4, dadurch gekennzeichnet, daß es eine oder mehrere der nachfolgenden Aminosäuresequenzen umfaßt: 
L-T-I-Q-V;
K-Y-E-N-Y-T;
L-M-D-P-K;
I-W-E-L-K;
V-M-S-Y-L-N-A;
(A)-V-S-N-M-L-Q-K;
N-A-S-I-S-V;
T-F-L-R;
D-I-I-K-P-D-P-P-K;
 
I-W-E-L-K-K-D-V-Y-V-V-E-L-D-W-Y-P-D-A-P-G-E-M;
und
X-N-L-P-V-A-(P oder X)-P-D-P-(S oder X oder
T)-M-F-P,
 wobei X eine beliebige Aminosäure darstellt.

6. Protein nach einem der Ansprüche 1 bis 5, das eines oder mehrere der nachfolgenden Kennzeichen aufweist:
(1) ein apparentes Molekulargewicht von ungefähr 70 bis 80 kD in der SDS-PAGE unter nichtreduzierenden Bedingungen;
(2) eine Untereinheit, die in der SDS-PAGE unter reduzierenden Bedingungen ein apparentes Molekulargewicht von ungefähr 40 kD hat;
(3) eine Untereinheit, die in der SDS-PAGE unter reduzierenden Bedingungen ein apparentes Molekulargewicht von ungefähr 30 bis 35 kD hat;
(4) ein isoelektrischer Punkt von 3,4 oder 4,8 in einem isoelektrischen Fokussiergel;
(5) Elution von einer Hydroxylapatit-Säule als ein einzelner Peak;
(6) Elution von einer Heparin-Sepharose-Säule als ein einzelner Peak;
(7) Elution von einer FPLC-Mono-Q®-Säule als ein einzelner Peak;
(8) biologische Aktivität in einem gamma-IFN-Induktionstest mit PBL;
(9) biologische Aktivität in einem GM-CSF-Induktionstest mit PBL;
(10) biologische Aktivität hinsichtlich der Aktivierung von NK-Zellen zur Abtötung von leukämie- und tumorabgeleiteten Zellen;
(11) biologische Aktivität in einem Tumor-Nekrose-Faktor-Induktionstest unter Verwendung von PHA-aktivierten T-Lymphozyten;
(12) co-mitogene Aktivität auf T-Lymphozyten aus peripherem Blut.

7. Protein nach einem der Ansprüche 1 bis 6, das man dadurch produziert, daß man ein konditioniertes Medium von RPMI 8866 einer aufeinanderfolgenden Reinigung durch eine QAE ZetaPrep®-Kartusche, eine Linsenlectin-Säule, eine Hydroxylapatit-Säule, eine Heparin-Sepharose-Säule und eine FPLC- (fast liquid chromatography-) Mono-Q®-Säule unterwirft, wobei das Protein von der letzteren Säule in einem einzelnen Peak eluiert.

8. Protein nach einem der Ansprüche 1 bis 6, das man dadurch produziert, daß man eine Zellinie züchtet, die mit einer das Protein codierenden DNA-Sequenz transformiert ist, wobei sich die DNA-Sequenz in einer funktionellen Verknüpfung mit einer Expressionskontroll-Sequenz befindet.

9. Protein nach einem der Ansprüche 1 bis 8, das eine spezifische Aktivität von mehr als 1x10⁷ Verdünnungseinheiten/mg in einem gamma-Interferon-Induktionstest aufweist.

10. Untereinheit eines Proteins nach einem der Ansprüche 1 bis 9.

11. Verfahren zur Herstellung eines homogenen Proteins nach einem der Ansprüche 1 bis 7, umfassend die nachfolgenden Schritte, die dadurch gekennzeichnet sind, daß man ein konditioniertes Medium von RPMI 8866 einer aufeinanderfolgenden Reinigung durch eine QAE ZetaPrep®-Kartusche, eine Linsenlectin-Säule, eine Hydroxylapatit-Säule, eine Heparin-Sepharose-Säule und eine FPLC- (fast liquid chromatography-) Mono-Q®-Säule unterwirft, wobei das Protein von der letzteren Säule in einem einzelnen Peak eluiert.

12. Verfahren nach Anspruch 11, ferner einen Schritt umfassend, der dadurch gekennzeichnet ist, daß man das Eluat der Mono-Q®-Säule einer Gelfiltrations-Chromatographie unterwirft.

13. Verfahren nach Anspruch 11 oder 12, dadurch gekennzeichnet, daß es gegebenenfalls eine Reinigung durch Reversphasen-HPLC vor der Gelfiltrations-Chromatographie einschließt.

14. DNA-Sequenz, die NKSF oder eine Untereinheit desselben codiert, umfassend eine DNA-Sequenz, die ausgewählt ist unter
(a) der DNA-Sequenz 
20                    40                    60
GAATTCCGTCGACTCTAGAGGCCCAGAGCAAGATGTGTCACCAGCAGTTGGTCATCTCTT
80                    100                   120
GGTTTTCCCTGGTTTTTCTGGCATCTCCCCTCGTGGCCATATGGAACTGAAGAAAGATG
 
140 180
TTTATGTCGTAGAATTGGATTGGTATCCGGAGGCCCCTGGAGAAATGGTGGTCCTCACCT
240
GTGACACCCCTGAAGAAGATGGTATCACCTGGACCTTGGACCAGAGCAGTGAGGTCTTAG
300
GCTCTGGCAAAACCCTGACCATCCAAGTCAAAGAGTTTGGAGATGCTGGCCAGTACACCT
360
GTCACAAAGGAGGCGAGGTTCTAAGCCATTCGCTCCTGCTGCTTCACAAAAAGGAAGATG
420
GAATTTGGTCCACTGATATTTTAAAGGACCAGAAAGAACCCAAAAATAAGACCTTTCTAA
480
GATGCGAGGCCAAGAATTATTCTGGACGTTTCACCTGCTGGTGGCTGACGACAATCAGTA
540
CTGATTTGACATTCAGTGTCAAAAGCAGCAGAGGCTCTTCTGACCCCCAAGGGGTGACGT
600
GCGGAGCTGCTACACTCTCTGCAGAGAGAGTCAGAGGGGACAACAAGGAGTATGAGTACT
660
CAGTGGAGTGCCAGGAGGACAGTGCCTGCCCAGCTGCTGAGGAGAGTCTGCCCATTGAGG
720
TCATGGTGGATGCCGTTCACAAGCTCAAGTATGAAAACTACACCAGCAGCTTCTTCATCA
780
GGGACATCATCAAACCTGACCCACCCAAGAACTTGCAGCTGAAGCCATTAAAGAATTCTC
840
GGCAGGTGGAGGTCAGCTGGGAGTACCCTGACACCTGGAGTACTCCACATTCCTACTTCT
900
CCCTGACATTCTGCGTTCAGGTCCAGGGCAAGAGCAAGAGAGAAAACTCTAGAGTCGACG

GAATTC
(b) DNA-Sequenzen, die mit der DNA-Sequenz unter (a) hybridisieren;
(c) allelischenVariationen, Analogen, Derivaten oder Fragmenten der DNA-Sequenz von (a) oder (b); oder
(d) DNA-Sequenzen, die sich wegen der Degeneriertheit des genetischen Codes in der Codonsequenz unterscheiden;
wobei besagtes NKSF oder ein die besagte Untereinheit enthaltendes Protein eine NKSF-Aktivität zeigen und in einem gamma-Interferon-Induktionstest aktiv sind.

15. DNA-Sequenz nach Anspruch 14, umfassend eine Sequenz von Nucleotiden, die eine oder mehrere der nachfolgenden Aminosäuresequenzen codiert: 
L-T-I-Q-V;

K-Y-E-N-Y-T;

L-M-D-P-K;

I-W-E-L-K;

V-M-S-Y-L-N-A;

(A)-V-S-N-M-L-Q-K;

N-A-S-I-S-V;

T-F-L-R;

D-I-I-K-P-D-P-P-K;

I-W-E-L-K-K-D-V-Y-V-V-E-L-D-W-Y-P-D-A-P-G-E-M;
und

X-N-L-P-V-A-(P oder X)-P-D-P-(S oder X oder T)-
M-F-P,
 wobei X eine beliebige Aminosäure darstellt.

16. DNA-Sequenz nach Anspruch 14, umfassend eine Sequenz von Nucleotiden, die die Aminosäuresequenz 
M-C-H-Q-
Q-L-V-I-S-W-F-S-L-V-F-L-A-S-P-L-V-A-I-W-E-L-K-K-D-V-Y-V-V-E-
L-D-W-Y-P-D-A-P-G-E-M-V-V-L-T-C-D-T-P-E-E-D-G-I-T-W-T-L-D-Q-
S-S-E-V-L-G-S-G-K-T-L-T-I-Q-V-K-E-F-G-D-A-G-Q-Y-T-C-H-K-G-G-
E-V-L-S-H-S-L-L-L-L-H-K-K-E-D-G-I-W-S-T-D-I-L-K-D-Q-K-E-P-K-
N-K-T-F-L-R-C-E-A-K-N-Y-S-G-R-F-T-C-W-W-L-T-T-I-S-T-D-L-T-F-
S-V-K-S-S-R-G-S-S-D-P-Q-G-V-T-C-G-A-A-T-L-S-A-E-R-V-R-G-D-N-
K-E-Y-E-Y-S-V-E-C-Q-E-D-S-A-C-P-A-A-E-E-S-L-P-I-E-V-M-V-D-A-
V-H-K-L-K-Y-E-N-Y-T-S-S-F-F-I-R-D-I-I-K-P-D-P-P-K-N-L-Q-L-K-
P-L-K-N-S-R-Q-V-E-V-S-W-E-Y-P-D-T-W-S-T-P-H-S-Y-F-S-L-T-F-C-
V-Q-V-Q-G-K-S-K-R-E-N-S-R-V-D-G-I
 codiert.

17. Vektor, umfassend eine DNA-Sequenz nach einem der Ansprüche 14 bis 16.

18. Vektor nach Anspruch 17, wobei die DNA-Sequenz funktionell mit (einer) Expressionskontrollsequenz(en) verknüpft ist.

19. Zelle, dadurch gekennzeichnet, daß sie mit dem Vektor nach Anspruch 17 oder 18 transformiert ist.

20. Zelle nach Anspruch 19, dadurch gekennzeichnet, daß sie eine Säugerzelle oder eine Bakterienzelle ist.

21. Verfahren zur Herstellung eines Proteins nach einem der Ansprüche 1 bis 6 oder einer Untereinheit davon nach Anspruch 10, umfassend das Züchten einer transformierten Zelle nach Anspruch 19 oder 20.

22. Arzneimittel, umfassend ein Protein nach einem der Ansprüche 1 bis 9 oder eine Untereinheit davon nach Anspruch 10 in einem pharmakologisch wirksamen Vehikel.

23. Arzneimittel nach Anspruch 22, das ferner ein zusätzliches Cytokin, Hämatopoetin, einen Wachstumsfaktor oder einen Antikörper umfaßt, der fähig ist, an den Fc-Teil von NK-Zellen zu binden.

24. Arzneimittel nach Anspruch 23, dadurch gekennzeichnet, daß das Cytokin IL-1, IL-2 oder IL-6 ist.

## Revendications

1. Protéine de type facteur de stimulation des cellules tueuses naturelles (NKSF), comprenant une sous-unité codée par une séquence d'ADN comprenant une séquence d'ADN choisie parmi
(a) la séquence d'ADN représentée dans le tableau I;
(b) des séquences d'ADN qui s'hybrident avec la séquence d'ADN de (a);
(c) des variantes allèles, analogues, dérivés ou fragments de la séquence d'ADN de (a) ou (b); ou
(d) des séquences d'ADN qui diffèrent par la séquence de codons, en raison de la dégénérescence du code génétique;
caractérisée en ce que la protéine NKSF contenant ladite sous-unité conserve l'activité NKSF et est active dans un essai d'induction d'interféron γ.

2. Protéine selon la revendication 1, dans laquelle ladite sous-unité a
(a) une masse moléculaire apparente d'environ 40 kDa dans l'électrophorèse SDS-PAGE dans des conditions réductrices; et
(b) la séquence N-terminale d'aminoacides suivante

I-W-E-L-K-K-D-V-Y-V-V-E-L-D-W-Y-P-D-A-P-G-E-M.

3. Protéine selon la revendication 2, dans laquelle ladite sous-unité comporte la séquence N-terminale d'aminoacides suivante 
M-C-H-Q-Q-L-V-I-S-W-

F-S-L-V-F-L-A-S-P-L-V-A-I-W-E-L-K-K-D-V-Y-V-V-E-L-D-W-Y-

P-D-A-P-G-E-M-V-V-L-T-C-D-T-P-E-E-D-G-I-T-W-T-L-D-Q-S-S-

E-V-L-G-S-G-K-T-L-T-I-Q-V-K-E-F-G-D-A-G-Q-Y-T-C-H-K-G-G-

E-V-L-S-H-S-L-L-L-L-H-K-K-E-D-G-I-W-S-T-D-I-L-K-D-Q-K-E-

P-K-N-K-T-F-L-R-C-E-A-K-N-Y-S-G-R-F-T-C-W-W-L-T-T-I-S-T-

D-L-T-F-S-V-K-S-S-R-G-S-S-D-P-Q-G-V-T-C-G-A-A-T-L-S-A-E-
 
R-V-R-G-D-N-K-E-Y-E-Y-S-V-E-C-Q-E-D-S-A-C-P-A-A-E-E-S-L-

P-I-E-V-M-V-D-A-V-H-K-L-K-Y-E-N-Y-T-S-S-F-F-I-R-D-I-I-K-

P-D-P-P-K-N-L-Q-L-K-P-L-K-N-S-R-Q-V-E-V-S-W-E-Y-P-D-T-W-

S-T-P-H-S-Y-F-S-L-T-F-C-V-Q-V-Q-G-K-S-K-R-E-N-S-R-V-D-G-

I,
 ou des fragments de celle-ci.

4. Protéine selon la revendication 1, comprenant une sous-unité ayant
(a) une masse moléculaire apparente d'environ 30 à 35 kDa dans l'électrophorèse SDS-PAGE dans des conditions réductrices; et
(b) la séquence N-terminale d'aminoacides
X-N-L-P-V-A-(P ou X)-P-D-P-(S ou X ou T)-M-F-P, X étant un aminoacide quelconque.

5. Protéine selon l'une quelconque des revendications 1 à 4, caractérisée en ce qu'elle comprend une ou plusieurs des séquences d'aminoacides suivantes: 
L-T-I-Q-V;
K-Y-E-N-Y-T;
L-M-D-P-K;
I-W-E-L-K;
V-M-S-Y-L-N-A;
(A)-V-S-N-M-L-Q-K;
N-A-S-I-S-V;
T-F-L-R;
D-I-I-K-P-D-P-P-K;
I-W-E-L-K-K-D-V-Y-V-V-E-L-D-W-Y-P-D-A-P-G-E-M; et
X-N-L-P-V-A-(P ou X)-P-D-P-(S ou X ou T)-M-F-P,
 X étant un aminoacide quelconque.

6. Protéine selon l'une quelconque des revendications 1 à 5, présentant une ou plusieurs des caractéristiques suivantes:
(1) une masse moléculaire apparente d'environ 70 à 80 kDa dans l'électrophorèse SDS-PAGE dans des conditions non réductrices;
(2) une sous-unité ayant une masse moléculaire apparente d'environ 40 kDa dans la SDS-PAGE dans des conditions réductrices;
(3) une sous-unité ayant une masse moléculaire apparente d'environ 30 à 35 kDa dans la SDS-PAGE dans des conditions réductrices;
(4) un point isoélectrique de 3,4 ou 4,8 dans un gel d'iso-électrofocalisation;
(5) l'élution sous forme d'un pic unique, à partir d'une colonne d'hydroxyapatite;
(6) l'élution sous forme d'un pic unique, à partir d'une colonne d'héparine-Sepharose;
(7) l'élution sous forme d'un pic unique, à partir d'une colonne de FPLC Mono-Q®;
(8) une activité biologique dans un essai d'induction d'IFN-γ par des lymphocytes de sang périphérique (LSP);
(9) une activité biologique dans un essai d'induction du facteur stimulant la formation de colonies de granulocytes/macrophages (GM-CSF) par des LSP;
(10) une activité biologique pour activer des cellules tueuses naturelles (NK) à tuer des cellules leucémiques et des cellules dérivées de tumeurs;
(11) une activité biologique dans un essai d'induction du facteur de nécrose tumorale au moyen de lymphocytes T activés par la PHA;
(12) une activité co-mitogène sur les lymphocytes T de sang périphérique.

7. Protéine selon l'une quelconque des revendications 1 à 6, produite par soumission d'un milieu conditionné, à partir de RPMI 8866, à une purification séquentielle à travers une cartouche QAE Zeta Prep®, une colonne de lectine de lentille, une colonne d'hydroxyapatite, une colonne d'héparine-Sepharose et une colonne Mono-Q® de chromatographie liquide rapide de protéine, ladite protéine étant éluée, sous forme d'un pic unique, de la dernière colonne.

8. Protéine selon l'une quelconque des revendications 1 à 6, produite par culture d'une lignée cellulaire transformée par une séquence d'ADN codant pour ladite protéine, ladite séquence d'ADN étant en association fonctionnelle avec une séquence régulatrice d'expression pour celle-ci.

9. Protéine selon l'une quelconque des revendications 1 à 8, ayant, dans un essai d'induction d'interféron γ, une activité spécifique de plus de 1×10⁷ unités de dilution/mg.

10. Sous-unité d'une protéine selon l'une quelconque des revendications 1 à 9.

11. Procédé pour la production d'une protéine homogène selon l'une quelconque des revendications 1 à 7, comprenant la soumission d'un milieu conditionné, à partir de RPMI 8866, à une purification séquentielle à travers une cartouche QAE Zeta Prep®, une colonne de lectine de lentille, une colonne d'hydroxyapatite, une colonne d'héparine-Sepharose et une colonne Mono-Q^{®} de chromatographie liquide rapide de protéine, ladite protéine étant éluée, sous forme d'un pic unique, de la dernière colonne.

12. Procédé selon la revendication 11, comprenant en outre la soumission de l'éluat de la colonne Mono-Q^{®} à une chromatographie par filtration sur gel.

13. Procédé selon la revendication 11 ou 12, comprenant éventuellement une purification par HPLC en phases inversées, avant ladite chromatographie par filtration sur gel.

14. Séquence d'ADN codant pour le NKSF ou une sous-unité de celui-ci, comprenant une séquence d'ADN choisie parmi
(a) la séquence d'ADN 
20                    40                    60
GAATTCCGTCGACTCTAGAGGCCCAGAGCAAGATGTGTCACCAGCAGTTGGTCATCTCTT
                    80                    100                   120
GGTTTTCCCTGGTTTTTCTGGCATCTCCCCTCGTGGCCATATGGGAACTGAAGAAAGATG
                    140                                         180
TTTATGTCGTAGAATTGGATTGGTATCCGGAGGCCCCTGGAGAAATGGTGGTCCTCACCT
                                                                240
GTGACACCCCTGAAGAAGATGGTATCACCTGGACCTTGGACCAGAGCAGTGAGGTCTTAG
                                                                300
GCTCTGGCAAAACCCTGACCATCCAAGTCAAAGAGTTTGGAGATGCTGGCCAGTACACCT
                                                                360
GTCACAAAGGAGGCGAGGTTCTAAGCCATTCGCTCCTGCTGCTTCACAAAAAGGAAGATG
                                                                420
GAATTTGGTCCACTGATATTTTAAAGGACCAGAAAGAACCCAAAAATAAGACCTTTCTAA
                                                                480
GATGCGAGGCCAAGAATTATTCTGGACGTTTCACCTGCTGGTGGCTGACGACAATCAGTA
                                                                540
CTGATTTGACATTCAGTGTCAAAAGCAGCAGAGGCTCTTCTGACCCCCAAGGGGTGACGT
                                                                600
GCGGAGCTGCTACACTCTCTGCAGAGAGAGTCAGAGGGGACAACAAGGAGTATGAGTACT
                                                                660
CAGTGGAGTGCCAGGAGGACAGTGCCTGCCCAGCTGCTGAGGAGAGTCTGCCCATTGAGG
                                                                720
TCATGGTGGATGCCGTTCACAAGCTCAAGTATGAAAACTACACCAGCAGCTTCTTCATCA
                                                                780
GGGACATCATCAAACCTGACCCACCCAAGAACTTGCAGCTGAAGCCATTAAAGAATTCTC
 
840
GGCAGGTGGAGGTCAGCTGGGAGTACCCTGACACCTGGAGTACTCCACATTCCTACTTCT
900
CCCTGACATTCTGCGTTCAGGTCCAGGGCAAGAGCAAGAGAGAAACTCTAGAGTCGACG
GAATTC
(b) des séquences d'ADN qui s'hybrident avec la séquence d'ADN de (a);
(c) des variantes allèles, analogues, dérivés ou fragments de la séquence d'ADN de (a) ou (b); ou
(d) des séquences d'ADN qui diffèrent par la séquence de codons, en raison de la dégénérescence du code génétique;
caractérisée en ce que le NKSF ou une protéine contenant ladite sous-unité manifestent une activité NKSF et sont actifs dans un essai d'induction d'interféron γ.

15. Séquence d'ADN selon la revendication 14, comprenant une séquence de nucléotides codant pour une ou plusieurs des séquences d'aminoacides suivantes: 
L-T-I-Q-V;
K-Y-E-N-Y-T;
L-M-D-P-K;
I-W-E-L-K;
V-M-S-Y-L-N-A;
(A)-V-S-N-M-L-Q-K;
N-A-S-I-S-V;
T-F-L-R;
D-I-I-K-P-D-P-P-K;
I-W-E-L-K-K-D-V-Y-V-V-E-L-D-W-Y-P-D-A-P-G-E-M; et
X-N-L-P-V-A-(P ou X)-P-D-P-(S ou X ou T)-M-F-P,
 X étant un aminoacide quelconque.

16. Séquence d'ADN selon la revendication 14, comprenant une séquence de nucléotides codant pour la séquence d'aminoacides

M-C-H-Q-Q-L-V-I-S-W-F-S-L-V-F-L-A-S-P-L-V-A-I-
W-E-L-K-K-D-V-Y-V-V-E-L-D-W-Y-P-D-A-P-G-E-M-V-V-L-T-C-D-
T-P-E-E-D-G-I-T-W-T-L-D-Q-S-S-E-V-L-G-S-G-K-T-L-T-I-Q-V-
K-E-F-G-D-A-G-Q-Y-T-C-H-K-G-G-E-V-L-S-H-S-L-L-L-L-H-K-K-
E-D-G-I-W-S-T-D-I-L-K-D-Q-K-E-P-K-N-K-T-F-L-R-C-E-A-K-N-
Y-S-G-R-F-T-C-W-W-L-T-T-I-S-T-D-L-T-F-S-V-K-S-S-R-G-S-S-
D-P-Q-G-V-T-C-G-A-A-T-L-S-A-E-R-V-R-G-D-N-K-E-Y-E-Y-S-V-
E-C-Q-E-D-S-A-C-P-A-A-E-E-S-L-P-I-E-V-M-V-D-A-V-H-K-L-K-
Y-E-N-Y-T-S-S-F-F-I-R-D-I-I-K-P-D-P-P-K-N-L-Q-L-K-P-L-K-
N-S-R-Q-V-E-V-S-W-E-Y-P-D-T-W-S-T-P-H-S-Y-F-S-L-T-F-C-V-
Q-V-Q-G-K-S-K-R-E-N-S-R-V-D-G-I.

17. Vecteur comprenant une séquence d'ADN selon l'une quelconque des revendications 14 à 16.

18. Vecteur selon la revendication 17, dans lequel ladite séquence d'ADN est fonctionnellement liée à une(des) séquence(s) régulatrice(s) d'expression.

19. Cellule transformée par le vecteur de la revendication 17 ou 18.

20. Cellule selon la revendication 19, qui est une cellule mammalienne ou bactérienne.

21. Procédé pour la production d'une protéine selon l'une quelconque des revendications 1 à 6 ou d'une sous-unité de celle-ci selon la revendication 10, comprenant la culture d'une cellule transformée conforme à la revendication 19 ou 20.

22. Composition pharmaceutique comprenant une protéine selon l'une quelconque des revendications 1 à 9 ou une sous-unité de celle-ci selon la revendication 10, dans un véhicule pharmaceutiquement acceptable.

23. Composition selon la revendication 22, comprenant en outre une cytokine, une hématopoïétine, un facteur de croissance ou un anticorps, supplémentaire, capable de liaison à la partie Fc de cellules NK.

24. Composition selon la revendication 23, dans laquelle ladite cytokine est IL-1, IL-2 ou IL-6.
